(19) Europäisches Patentamt
European Patent Office
Office européen des brevets

(11) EP 0 358 991 B2

(12) NEUE EUROPÄISCHE PATENTSCHRIFT

(45) Veröffentlichungstag und Bekanntmachung des Hinweises auf die Entscheidung über den Einspruch:
**11.02.1998 Patentblatt 1998/07**

(45) Hinweis auf die Patenterteilung:
**23.02.1994 Patentblatt 1994/08**

(21) Anmeldenummer: **89115557.4**

(22) Anmeldetag: **23.08.1989**

(51) Int Cl.[6]: **G01N 33/84**, G01N 33/52, G01N 31/22

(54) **Testvorrichtung zur optischen Bestimmung von Kationen, Anionen oder elektrisch neutralen ionogenen Spezien und Testverfahren unter Verwendung dieser Testvorrichtung**

Test device for an optical determination of cations, anions or electrically neutral ionogenic species, and test method using said device

Dispositif de test pour la détermination optique de cations, d’anions ou de substances ionogènes électriquement neutres et procédé de test utilisant ce dispositif

(84) Benannte Vertragsstaaten:
**AT BE CH DE ES FR GB IT LI LU NL SE**

(30) Priorität: **26.08.1988 CH 3175/88**

(43) Veröffentlichungstag der Anmeldung:
**21.03.1990 Patentblatt 1990/12**

(73) Patentinhaber: **Willi Möller AG**
**CH-8050 Zürich (CH)**

(72) Erfinder:
- **Simon, Wilhelm**
  **CH-8006 Zürich (CH)**
- **Seiler, Kurt**
  **CH-8409 Winterthur (CH)**
- **Morf, Werner Erich**
  **CH-8049 Zürich (CH)**

(74) Vertreter: **Blum, Rudolf Emil Ernst et al**
**c/o E. Blum & Co**
**Patentanwälte**
**Vorderberg 11**
**8044 Zürich (CH)**

(56) Entgegenhaltungen:
**EP-A- 125 555**
**EP-A- 0 010 615**
**EP-A- 0 153 641**
**EP-A- 0 281 829**
**US-A- 4 714 763**

- **CHIMIA, Band 41, Nr. 11, November 1987, Basel (CH); C.BEHRINGER et al., Seiten 397-398#**
- **ANALYTICAL CHEMISTRY, Band 59, 1987, American Chemical Society, Washington, DC (US); M.E. MEYERHOFF et al., Seiten 144-150#**
- **BIOELECTROANALYTICAL SYMPOSIUM, 1986, Matrafüred, HU; W. SIMON et al., Seiten 173-177#**
- **JOURNAL OF THE CHEMICAL SOCIETY, FARADAY TRANS. 1, Band 82, 1986, London (GB); U. OESCH et al., Seiten 1179-1186#**
- **CHIMIA, Band 33, Nr. 12, Dezember 1979, Basel (CH); W.E. MORF et al., Seiten 452-457#**
- **Packungs Beilage eines "SERALYZER" test der AMES Division von Miles Laboratories, 1985**
- **The Merck Index, 10. Auflage, 1983, Referenznummer 3637, Seiten 533-534**

EP 0 358 991 B2

**Beschreibung**

HINTERGRUND DER ERFINDUNG

Ziel der vorliegenden Erfindung war es, eine Testvorrichtung zu entwickeln, mit deren Hilfe die Anwesenheit, beziehungsweise Konzentration, beziehungsweise Aktivität von Kationen, Anionen oder elektrisch neutralen ionogenen Spezien in einer im wesentlichen wässrigen Probelösung durch eine Aenderung der optischen Eigenschaften dieser Testvorrichtung feststellbar ist. Die Aenderung der optischen Eigenschaften kann eine Ausbildung oder eine Löschung einer Fluoreszenz, eine Veränderung der Lichtabsorption im ultravioletten Bereich, im sichtbaren Bereich oder im infraroten Bereich sein.

Aus der Literatur sind viele Methoden zur Bestimmung von Ionen in wässrigen, beziehungsweise im wesentlichen wässrigen Probelösungen bekannt, wie zum Beispiel die Bestimmung derselben mit Hilfe von Flammenphotometrie, Atomabsorptionsspektrophotometrie oder Teststreifen.

Kationspezifische Einzelelektroden, deren ionenselektiver Teil einen kationselektiven lipophilen Ionophor enthält, beziehungsweise anionspezifische Einzelelektroden, deren ionenselektiver Teil einen anionselektiven lipophilen Ionophor enthält, werden seit vielen Jahren zur elektrometrischen Bestimmung der Aktivität, beziehungsweise Konzentration von Kationen oder Anionen in wässrigen Probelösungen herangezogen. Bei der Durchführung der Bestimmung kommt die Oberfläche des anionselektiven Teils, beispielsweise die anionselektive Membran einer entsprechenden anionselektiven Elektrode mit der Probelösung in Berührung und an dieser Oberfläche reagiert der in der Membran enthaltene anionselektive Ionophor mit dem zu bestimmenden Anion der Probelösung, wodurch ein Membranpotential hervorgerufen wird, welches sich in Abhängigkeit von der Konzentration des zu bestimmenden Anions in der Probelösung ändert. In der Regel wird nur ein geringer Prozentsatz des anionselektiven Ionophors in den Oberflächenbereichen der anionselektiven Membran in den entsprechenden Anionkomplex übergeführt, während die Hauptmenge des anionselektiven lipophilen Ionophors als unkomplexierter neutraler anionselektiver Ionophor in dem Körper der Membran enthalten bleibt. Das Ausmass der Komplexbildung in derartigen anionselektiven Membranen von anionselektiven Einzelelektroden ist also sehr gering und in manchen Fällen sogar nahezu unabhängig von der Konzentration des zu bestimmenden Anions in der wässrigen Probelösung, dennoch werden bei entsprechenden potentiometrischen Bestimmungen von Anionen mit anionselektiven Einzelelektroden im allgemeinen sehr gute Ergebnisse erzielt, weil die Aenderung des Membranpotentials der Aenderung der Konzentration, beziehungsweise Aktivität des zu bestimmenden Anions in der Probelösung entspricht.

Analog sind die Verhältnisse bei kationspezifischen Einzelelektroden, deren ionenselektive Membran einen der vielen in der Literatur beschriebenen kationselektiven lipophilen Ionophore enthält.

Es wurde auch bereits versucht, anionselektive lipophile Ionophore zu entwickeln, die in ihrem Molekül einen chromphore Gruppe aufweisen, welche die optischen Eigenschaften des Ionophors ändert, sobald dieser mit dem zu bestimmenden Anion einen Komplex bildet. Wenn sich der entsprechende, eine chromophore Gruppe aufweisende anionselektive Ionophor gelöst oder dispergiert in einem im wesentlichen hydrophoben Trägermaterial befindet, dann tritt bei dem Kontakt mit der Probelösung nur in den äussersten Schichten der Oberfläche die Komplexbildung zwischen dem anionselektiven Chromoionophor und den zu bestimnenden Anionen der Probelösung auf und die Messergebnisse sind dementsprechend unbefriedigend.

Analoges gilt für kationselektive Testvorrichtungen auf Basis eines Trägermaterials und eines kationselektiven Chromoionophors.

Es wurde jetzt überraschenderweise festgestellt, dass die erwähnten Nachteile dadurch beseitigt werden können, dass man gewährleistet, dass dann, wenn das den anionselektiven Ionophor enthaltende Trägermaterial mit der die zu bestimmenden Anionen enthaltenden wässrigen Probelösung in Berührung kommt, ein Austausch von im Trägermaterial enthaltenen Anionen gegen Anionen der wässrigen Probelösung stattfindet, oder eine Coextraktion von Anionen und Kationen aus der wässrigen Probelösung in das Trägermaterial der Testvorrichtung erfolgt.

In gleicher Weise muss auch bei der Bestimmung von Kationen mit Hilfe einer Testvorrichtung auf Basis eines Trägermaterials und eines kationselektiven Ionophors gewährleistet werden, dass ein Austausch von Kationen der Testvorrichtung gegen die zu bestimmenden Kationen der wässrigen Probelösung stattfindet, beziehungsweise eine Coextraktion von Kationen und Anionen aus der Probelösung in die Testvorrichtung stattfindet.

Es zeigte sich, dass dieser Ionenaustausch, beziehungsweise die Coextraktion von Anionen und Kationen im Falle der anionselektiven Testvorrichtungen erfindungsgemäss erreicht werden kann, indem man in diese Testvorrichtungen zusätzlich zu dem anionselektiven Ionophor noch mindestens eine weitere Komponente einverleibt, die beim Kontakt der Testvorrichtung mit der Probelösung eine simultane Coextraktion von Anionen und Kationen der Probelösung oder die Extraktion einer elektrisch neutralen ionogenen Spezies der Probelösung in die Testvorrichtung bewirkt, oder durch die ein Austausch von Anionen der Probelösung gegen eine äquivalente Menge von in der Testvorrichtung gebundenen Anionen auftritt.

In analoger Weise muss eine Testvorrichtung zur Bestimmung von Kationen auf Basis eines Trägermaterials und

eines kationselektiven lipophilen Ionophors erfindungsgemäss ebenfalls noch eine weitere Komponente enthalten, die gewährleistet, dass beim Kontakt der Testvorrichtung mit der Probelösung eine simultane Coextraktion von Kationen und Anionen der Probelösung oder die Extraktion einer elektrisch neutralen ionogenen Spezies der wässrigen Probelösung in die Testvorrichtung und/oder ein Austausch von Kationen der Probelösung gegen eine äquivalente Menge von in der Testvorrichtung vorhandenen Kationen erfolgt.

Damit ferner erreicht wird, dass die Bestimmung der Anionen, beziehungsweise Kationen, in der wässrigen Probelösung mit Hilfe von optischen Methoden durchführbar ist, muss in diesen mindestens ein kationselektiver lipophiler Ionophor und/oder mindestens ein anionselektiver lipophiler Ionophor und/oder mindestens eine weitere Komponente eine chromophore Gruppe aufweisen, die in der Lage ist, die optischen Eigenschaften des entsprechenden Ionophoren oder der Komponente zu ändern, sobald der entsprechende Ionophor oder die entsprechende Komponente mit Anionen, beziehungsweise Kationen, beziehungsweise einer entsprechenden elektrisch neutralen ionogenen Spezies der Probelösung in Berührung kommt.

Die erfindungsgemässen Testvorrichtungen, beispielsweise Teststreifen oder selektive Teile von Optoden, ermöglichen somit eine rasche, verlässliche und einfache Bestimmung von Anionen, Kationen, beziehungsweise elektrisch neutralen ionogenen Spezien durch eine Aenderung der optischen Eigenschaften dieser Vorrichtung.

## BESCHREIBUNG DES STANDES DER TECHNIK

Wie bereits erwähnt wurde, werden seit vielen Jahren kationspezifische Einzelelektroden oder anionspezifische Einzelelektroden zur Bestimmung der Anwesenheit, beziehungsweise Konzentration, beziehungsweise Aktivität von Kationen oder Anionen in wässrigen Lösungen herangezogen. Die ionenempfindlichen Teile dieser ionenempfindlichen Elektroden enthalten einen kationselektiven lipophilen Ionophor oder einen anionselektiven lipophilen Ionophor, der eine Selektivität gegenüber den in der Probelösung zu bestimmenden Kationen, beziehungsweise Anionen aufweist.

Wenn der kationselektive Teil, beispielsweise eine kationselektive Membran einer entsprechenden kationselektiven Elektrode, mit der Probelösung, die das zu bestimmende Kation enthält, in Berührung kommt, dann reagiert ein Teil des kationselektiven Ionophors mit dem entsprechenden Kation, wodurch ein Membranpotential hervorgerufen wird, welches der Gleichgewichtsverteilung der ausgewählten Ionen zwischen dem Körper der Membran und der sie aussen berührenden Probelösung, sowie der sie innen berührenden Elektrodenfüllösung entspricht. Im Idealfall zeigen derartige kationselektiven Membranelektodenhalbzellen eine reversible Response auf die Einzelionenaktivität des entsprechenden Kations in der Probelösung gemäss der Nernst'schen Gleichung, sodass die Einzelionenaktivität durch die Messung des elektrischen Potentials, also der EMK, wie folgt bestimmt werden kann:

$$EMK = \frac{RT}{nF} \ln \frac{a_1}{a_2} .$$

In dieser Nernst'schen Gleichung haben die Symbole die folgende Bedeutung:

EMK = die elektromotorische Kraft des Systems, ausgedrückt in Volt.
F = Faraday-Konstante [96.487 Joule (Volt Aequiv.)$^{-1}$]
R = Gaskonstante [8,3144 Joule (mol Kelvin)$^{-1}$]
T = Temperatur in °K ln = natürlicher Logarithmus
$a_1$ = Aktivität des zu bestimmenden Ions in der Probelösung
$a_2$ = Aktivität des zu bestimmenden Ions in der Elektrodenfüllung
n = Ladungszahl des zu bestimmenden Ions, das an der Komplexbildungsreaktion mit dem ionenselektiven lipophilen Ionophor beteiligt ist [Aequiv. mol$^{-1}$]

Analog sind die Verhältnisse bei anionselektiven Elektroden, in denen der ionenempfindliche Teil einen anionselektiven lipophilen Ionophor enthält, der in der Lage ist, selektiv mit den zu bestimmenden Anionen einen Komplex auszubilden.

In den entsprechenden ionenempfindlichen Membranen ist der entsprechende lipophile kationselektive Ionophor, beziehungsweise der entsprechende lipophile anionselektive Ionophor, im allgemeinen in einem im wesentlichen hydrophoben Polymermaterial, häufig gelöst in einer Weichmacherkomponente, enthalten. Wie bereits erwähnt wurde, wird bei der Durchführung der Bestimmung, also bei Kontakt der ionenempfindlichen Membran mit der Probelösung in der Regel nur ein geringer Prozentsatz dieses Ionophors in den entsprechenden Kationkomplex, beziehungsweise Anionkomplex übergeführt, während die Hauptmenge des Ionophors als neutraler lipophiler Ionophor vorhanden bleibt. Die Ergebnisse entsprechender Untersuchungen, die anhand von kaliumselektiven Membranen durchgeführt wurden, welche Valinomycin als kaliumselektiven Ionophor in einem Polyvinylchloridträger enthalten, sind in der Veröffentlichung von A. Thoma et al., Analytical Chemistry, Band 49, Seite 1567, 1977, beschrieben.

In derartigen kationselektiven Teilen von kationselektiven Membranen ist dementsprechend das Ausmass der Komplexbildung zwischen dem zu bestimmenden Kation und dem kationselektiven lipophilen Ionophor im Vergleich zu dem in dem ionoselektiven Teil enthaltenen lipophilen Ionophor sehr gering und/oder sogar nahezu unabhängig von der Konzentration des zu bestimmenden Kations in der wässrigen Probelösung, die mit der einen Seite des fraglichen ionenempfindlichen Teils der ionenempfindlichen Elektrode in Berührung steht. Eine sehr gute Genauigkeit und Reproduzierbarkeit der Bestimmung der Aktivität des fraglichen Kations in der Probelösung ist dennoch gewährleistet, weil, so wie aus der oben angegebenen Nernst'schen Gleichung ersichtlich ist, durch die Messung der EMK das Aktivitätsverhältnis des fraglichen Kations in der Probelösung im Verhältnis zu der entsprechenden Aktivität in der Elektrodenfülllösung bestimmt wird und mit Hilfe von Eichkurven die Aktivität, beziehungsweise Konzentration des zu bestimmenden Kations, beziehungsweise Anions in der Probelösung direkt ablesbar ist.

Es sind eine grosse Anzahl an kationselektiven lipophilen Ionophoren bekannt, die selektiv mit unterschiedlichen Arten von Kationen Komplexe bilden und die als kationselektive Komponente der entsprechenden kationselektiven Teile von kationselektiven Elektroden eingesetzt werden können. Es sei in diesem Zusammenhang beispielsweise auf entsprechende Dicarbonsäurediamide verwiesen, die in der USA Patentschrift Nr. 3 957 607 von Simon et al. beschrieben sind. Das gleiche gilt auch für anionselektive lipophile Ionophore. Es sei in diesem Zusammenhang beispielsweise auf die Ketoverbindungen verwiesen, die selektiv mit Anionen von Oxasäuren in Wechselwirkung treten, die in der nicht zum Stande der Technik gehörenden europäischen Patentveröffentlichung Nr. 0 281 829 der Firma Willi Möller, die am 14. September 1988 veröffentlicht werden wird, beschrieben sind.

Des weiteren werden in der Veröffentlichung von Greenberg et al. in Analytica Chimica Acta, 141 (1982) Seiten 57 - 64 carbonatselektive Elektroden beschrieben, die als carbonatselektive Komponente Trifluoracetyl-p-butylbenzol enthalten, und zwar ist dieses carbonatselektive Keton in einer Polyvinylchloridmembran zusammen mit einem Esterweichmacher und allenfalls zusammen mit dem quaternären Ammoniumsalz Tricaprylylmethylammoniumchlorid enthalten. Carbonatselektive Membranelektroden, welche den gleichen carbonatselektiven lipophilen Ionophor, gegebenenfalls ebenfalls zusammen mit Tricaprylylmethylammoniumchlorid enthalten, bei denen jedoch das Polymermaterial Celluloseacetat ist, sind ferner in der Veröffentlichung von Herman in Analytica Chimica Acta, 76 (1975), Seiten 155 - 164 beschrieben.

Carbonatselektive Flüssigmembranelektroden, welche die gleiche carbonatselektive Komponente und ausserdem das quaternäre Ammoniumsalz Tridodecylmethylammoniumchlorid in einer Polyvinylchloridmembran enthalten, werden ferner in der Veröffentlichung von Meyerhoff et al. in Analytical Chemistry, Band 59, Nr. 1, Januar 1987, Seiten 144 - 150 beschrieben und dort wurde auch festgestellt, dass bei steigendem Gehalt an dem quaternären Ammoniumsalz die Carbonatempfindlichkeit gesteigert werden kann. Nicht erkannt wurde jedoch in dieser Veröffentlichung, dass die Anwesenheit des Tridodecylmethylammoniumchlorides in der Polyvinylmembran dazu führt, dass die Chloridionen dieses quaternären Ammoniumsalzes bei Kontakt mit der carbonationenenthaltenden wässrigen Probelösung gegen die Carbonationen der wässrigen Probelösung ausgetauscht werden.

Die europäische Patentveröffentlichung 0 010 615 betrifft ein Verfahren zur Bestimmung von Ionen oder polaren und/oder lipophilen Substanzen in Flüssigkeiten, bei welchem man diese zu bestimmenden Substanzen auf einen gegenüber diesen selektiven Komplexliganden oder ein selektives Wirtsmolekül einwirken lässt, und wobei dieser, beziehungsweise dieses durch eine direkte covalente oder heteropolare oder Wasserstoffbrücken- oder hydrophobe Bindung mit einem Chromophor verknüpft ist oder den Chromophor in Form eines Einschlusskomplexes enthält. Bei dieser Bestimmung wird eine Extinktionsänderung oder eine Wellenlängenverschiebung gemessen. Als Chromophor werden Farbstoffe, Fluoreszenzfarbstoffe oder Chromogene eingesetzt und als Komplexligand insbesondere solche aus der Gruppe der Kronenäther oder der kationselektiven Makrolide, beziehungsweise kationselektiven Antibiotica, wie Valinomycin, verwendet.

Speziell beschrieben sind in dieser europäischen Patentveröffentlichung Kronenäther, von denen ein oder zwei Mole an den Rest eines Farbstoffes, beispielsweise eines Azofarbstoffes, gebunden sind, sodass bei der Reaktion dieser ionenselektiven Komponente mit dem zu bestimmenden Ion eine Farbverschiebung auftritt. Des weiteren sind dort auch optische Bestimmungen von Kaliumionen auf Basis eines Komplexes von Valinomycin mit Farbstoffkationen beschrieben, wobei die Farbreaktion darauf beruht, dass die Kaliumionen in das Valinomycin eingelagert werden und das Farbstoffkation aus dem Komplex verdrängen (siehe Seite 18, Zeile 24 bis Seite 19, Zeile 22).

Gemäss den Beispielen werden die entsprechenden ionenselektiven Farbstoffe direkt zur photometrischen Bestimmung der Ionen herangezogen. Gemäss Beispiel 2 befindet sich der kaliumselektive Farbstoff auf einem porösen Trägermaterial, nämlich einem Filterpapierstreifen. In Anspruch 20 werden noch andere verwendbare organische oder anorganische Trägermaterialien genannt. Nirgends ist jedoch in dieser europäischen Patentveröffentlichung ein Hinweis zu finden, dass in dem Trägermaterial noch eine weitere Komponente vorhanden sein soll, die eine Ueberführung von Gegenionen zu dem bestimmenden Ion aus der Testlösung in das Trägermaterial bewirkt.

Es wurde auch bereits versucht, kationselektive lipophile Ionophore, beziehungsweise anionselektive lipophile Ionophore zu entwickeln, wobei die entsprechenden Ionophore sich in einem oder auf einem Trägermaterial einer

Testvorrichtung befinden und die fraglichen Ionophore eine chromophore Gruppe aufweisen, die dann, wenn eine Komplexbildung zwischen dem kationselektiven lipophilen Ionophor und dem zu bestimmenden Kation, beziehungsweise dem lipophilen anionselektiven Ionophor und dem zu bestimmenden Anion auftritt, eine Aenderung der optischen Eigenschaften dieses Ionophors hervorruft. Entsprechende anionselektive, die Farbe verändernden Ionophore sind beispielsweise in der nicht zum Stande der Technik gehörenden oben erwähnten europäischen Patentveröffentlichung Nr. 0 281 829 beschrieben.

In der Veröffentlichung von C. Behringer, B. Lehmann und W. Simon in CHIMIA, Band 41, Nr. 11, 1987, Seiten 397 und 398 werden ferner carbonatselektive Chromoionophore beschrieben, bei denen eine mit einer Trifluormethylgruppe substituierte Ketogruppe an einen Benzolkern gebunden ist, und die in Parastellung dazu eine Arylazogruppe als chromophore Gruppe tragen. Bei diesen carbonatselektiven Chromoionophoren wurde festgestellt, dass diese, gelöst in einem organischen Lösungsmittel, wie Aceton, Dioxan, Diäthyläther oder Hexan, dann eine Farbverschiebung in Richtung zu einer Absorption bei kürzeren Wellenlängen (hypsochrome Verschiebung) aufweisen, wenn in dieses organische Lösungsmittel ein darin gut lösliches Carbonat mit stark lipophilen Eigenschaften, nämlich Bis(tridodecylmethylammonium)carbonat eingebracht wird. In dieser Veröffentlichung wird ferner erwähnt, dass es früher schon bekannt war, dass Polymermembranen, welche solche Trifluoracetophenone als carbonatselektive Komponente enthalten, die keine chromophore Gruppe aufweisen, dann bei einer potentiometrischen Bestimmung der Carbonationen eine hohe Selektivität gegenüber diesen aufweisen, falls diese Polymermembranen ausserdem noch einen klassischen Anionenaustauscher enthalten (siehe Seite 397, linkeste Spalte, Zeilen 1 - 8 des zweiten Absatzes). In Analogie zu diesen früheren Untersuchungen wurden auch in der erwähnten Veröffentlichung potentiometrische Untersuchungen an Polymermembranen durchgeführt, die jedoch jetzt als carbonatselektive Komponente einen bestimmten der dort beschriebenen Chromoionophore enthielten, nämlich diejenige Verbindung, in der in der Parastellung zu der Trifluormethylgruppe eine solche Arylazogruppe gebunden ist, die selbst in der Parastellung zu ihrer Azogruppe eine n-Dodecyläthergruppierung als Substituenten trägt. Diese potentiometrischen Studien wurden anhand solcher Polymermembranen durchgeführt, die 3,9 Gew.-% an diesem carbonatselektiven Chromoionophor, 40,4 Gew.-% an Polyvinylchlorid, 53,7 Gew.-% an einem Weichmacher, und ausserdem 2,0 Gew.-% an einem lipophile Eigenschaften aufweisenden Chlorid enthielten, nämlich an Tridodecyl-methyl-ammoniumchlorid. Dabei stellte es sich heraus, dass bei den potentiometrischen Untersuchungen die Membranen ein optimales Ansprechen auf Carbonationen bereits bei diesem sehr geringen Gehalt an dem erwähnten Chromoionophor aufweisen, während analog aufgebaute Membranen, die ein entsprechendes Trifluorcacetophenon ohne chromophoren Rest enthalten (nämlich 1-Butyl-4-trifluoracetylbenzol), erst bei einem Gehalt von 14,2 Gew.-% an dieser carbonatselektiven Componente ein optimales Ansprechen bei den potentiometrischen Untersuchungen aufweisen (siehe Seite 397, rechteste Spalte, vorletzter Absatz).

Obwohl also in dieser Veröffentlichung Polymermembranen beschrieben sind, die zusätzlich zu dem speziellen carbonatselektiven Chromoionophor noch ein lipophiles Chlorid enthalten, das also prinzipiell zu einem Austausch der Chloridanionen der Polymermembran gegen Anionen aus der Probelösung geeignet wäre, so wurde dennoch die entsprechende Membran nicht als Membran zur optischen Bestimmung von Carbonatanionen herangezogen, sondern nur zur potentiometrischen Bestimmung von Carbonatanionen, und es wird ferner auch bezüglich der den Chromoionophor enthaltenden Polymermembran nicht erwähnt, dass bei dieser eine Farbverschiebung beobachtet wurde, sobald diese Membran mit einer wässrigen, Carbonatanionen enthaltenden Lösung in Kontakt gebracht wurde.

Bei der Reaktion eines Kations der Probelösung mit einem in einem geeigneten Trägermaterial, zum Beispiel Polyvinylchlorid, befindlichen kationselektiven Ionophor, werden vom Trägermaterial Protonen abgegeben. Es sei in diesem Zusammenhang auf die bereits oben erwähnte Veröffentlichung von A. Thoma et al. und die Veröffentlichung von W.E. Morf et al., Chimia 33, Seite 452, 1979, verwiesen. Es ist somit möglich, die entsprechende Komplexbildung mit dem Ionophor in einem entsprechenden Teststreifen mit Hilfe eines pH-Indikators optisch anzuzeigen. Entsprechende kaliumselektive Teststreifen, die als kaliumselektiven Ionophor Valinomycin enthalten, und bei denen in der organischen Phase des Teststreifens aus Papier die folgende Reaktion abläuft:

$$K^{+} + \text{Ionophor} + \text{H-Farbstoff} \rightleftarrows [\text{K-Ionophor}]^{+} + [\text{Farbst.}]^{-} + H^{+}$$

sind bereits beschrieben (siehe die kaliumselektiven Teststreifen mit der Bezeichnung Seralyzer).

In der europäischen Patentveröffentlichung Nr. 0 153 641 der Firma Miles Laboratories sind Testvorrichtungen zur Bestimmung der Anwesenheit eines Ions in wässrigen Testlösungen beschrieben. Diese Testvorrichtungen weisen ein poröses Trägermaterial auf, in das im wesentlichen gleichmässig die folgenden Komponenten einverleibt sind:

a) eine homogene hydrophobe Zusammensetzung, die einen Ionophoren enthält, der in der Lage ist, einen Komplex mit dem Ion zu bilden, sowie ein hydrophobes Vehikel und eine Hilfssubstanz, die in der Lage ist, mit dem Komplex aus dem Ionophor und dem Ion in Wechselwirkung zu treten, wobei eine feststellbare Veränderung dieser Hilfssubstanz auftritt und ferner

b) ein Puffer, der einen pH-Wert im Bereich von etwa 5 - 10 aufrecht erhält.

Die dort verwendeten Hilfssubstanzen sind vorzugsweise substituierte Phenole, bei denen eine Farbe oder Farbveränderung oder eine Fluoreszenz gebildet wird, sobald sie mit dem Komplex aus Ionophor und Ion in Anwesenheit des Puffers in Wechselwirkung treten.

Die bisher bekannten Testvorrichtungen auf Basis eines ionenselektiven lipophilen Ionophors, der eine chromophore Gruppe aufweist, die die optischen Eigenschaften des Ionophors ändert, sobald dieser mit dem zu bestimmenden Ion der Probelösung einen Komplex bildet und auch die Testvorrichtung auf Basis eines lipophilen Ionophors in Kombination mit einer Testsubstanz, bei der dann, wenn diese mit dem Komplex aus Ionophor und Ion in Wechselwirkung tritt, eine Aenderung einer optischen Eigenschaft feststellbar ist, beispielsweise ein Indikator, weisen jedoch meistens den bereits weiter vorne erwähnten Nachteil auf, dass in dem Trägermaterial in der Regel nur ein sehr geringer Teil des vorhandenen Ionophors mit dem Ion einen Komplex bildet und dementsprechend die optische Veränderung entweder zu gering ist um feststellbar zu sein und/oder sogar nahezu unabhängig von der Konzentration des zu bestimmenden Ions in der Probelösung ist.

Es wurde bisher versucht, diesen Nachteil dadurch zu beseitigen, dass ein poröses Trägermaterial angewandt wird, um genügend grosse Oberflächen zur Verfügung zu stellen, an denen die Komplexbildung zwischen dem lipophilen Ionophor und dem zu bestimmenden Ion hervorgerufen werden kann (siehe die porösen Trägermaterialien der Testvorrichtung, die in der weiter vorner erwähnten europäischen Patentveröffentlichung 0 153 641 beschrieben sind und die Teststreifen mit der Bezeichnung Seralyser). Durch die Verwendung von porösen Trägermaterialien konnte jedoch der Nachteil nicht beseitigt werden, dass allenfalls das Ausmass der Komplexbildung zwischen dem ionenselektiven Ionophor und dem zu bestimmenden Ion von der Konzentration dieses Ions in der Probelösung nahezu unabhängig ist, oder zumindestens das Ausmass der Komplexbildung nicht proportional mit der Konzentration des zu bestimmenden Ions in der Probelösung ansteigt.

Es wurde nun überraschenderweise festgestellt, dass diese Nachteile von bisher bekannten Testvorrichtungen zur Bestimmung der Konzentration, beziehungsweise Aktivität von Kationen oder Anionen in im wesentlichen wässrigen Probelösungen auf Basis von kationselektiven lipophilen Ionophoren, beziehungsweise anionselektiven lipophilen Ionophoren, die eine chromophore Gruppe aufweisen oder auf Basis von entsprechenden kationselektiven lipophilen Ionophoren, beziehungsweise anionselektiven lipophilen Ionophoren, in Kombination mit einer Hilfssubstanz, die bei der Komplexbildung zwischen dem Ionophor und dem entsprechenden Ion die optischen Eigenschaften ändert, vermieden werden können, indem man gewährleistet, dass bei Berührung der Testvorrichtung mit der Probelösung simultan Anionen und Kationen aus der Probelösung in die Testvorrichtung übergehen und/oder Kationen der Probelösung gegen Kationen der Testvorrichtung, beziehungsweise Anionen der Probelösung gegen Anionen der Testvorrichtung ausgetauscht werden, beziehungsweise elektrisch neutrale ionogene Spezien der Probelösung gegen elektrisch neutrale ionogene Spezien der Testvorrichtung ausgetauscht werden.

Durch diese neuartige Testvorrichtung wird gewährleistet, dass in Abhängigkeit von der Aktivität beziehungsweise der Konzentration des Ions, beziehungsweise der ionogenen Spezies in der Probelösung unterschiedliche Mengen des ionenselektiven lipophilen Ionophors in der Testvorrichtung komplexiert werden und dementsprechend die Aenderung der optischen Eigenschaften des Ionophors und/oder einer weiteren Komponente der Testvorrichtung, welche eine chromophore Gruppe aufweist. von der zu bestimmenden Konzentration des Ions in der Probelösung abhängig ist.

## BESCHREIBUNG DER ERFINDUNG

Ein Gegenstand der vorliegenden Erfindung ist eine Testvorrichtung zur optischen Bestimmung der Anwesenheit, beziehungsweise Konzentration, beziehungsweise Aktivität von Kationen, Anionen oder elektrisch neutralen ionogenen Spezien in einer im wesentlichen wässrigen Probelösung. wobei diese Testvorrichtung in einem im wesentlichen nicht porösen Trägermaterial, welches ein Polymermaterial ist, das nur geringe Anteile an hydrophilen Gruppen aufweist oder frei von hydrophilen Gruppen ist, mindestens einen kationselektiven lipophilen Ionophor und/oder mindestens einen anionselektiven lipophilen Ionophor enthält, und diese Testvorrichtung dadurch gekennzeichnet ist. dass das Trägermaterial ausserdem eine weitere Komponente enthält, durch welche bei dem Kontakt der Testvorrichtung mit der wässrigen Probelösung ein simultaner Uebergang von Anionen und Kationen aus der wässrigen Probelösung in die Testvorrichtung und/oder ein Austausch von Kationen, beziehungsweise Anionen der Probelösung gegen in der Testvorrichtung enthaltene Kationen, beziehungsweise Anionen, beziehungsweise ein Austausch elektrisch neutraler ionogener Spezien der wässrigen Probelösung gegen elektrisch neutrale ionogene Spezien der Testvorrichtung gewährleistet ist, und wobei dieses Trägermaterial entweder

A) mindestens einen kationselektiven lipophilen Ionophor und ausserdem mindestens einen anionselektiven lipophilen Ionophor enthält, wobei mindestens einer dieser Ionophore eine chromophore Gruppe aufweist, die in der Lage ist, ihre optischen Eigenschaften zu ändern, sobald dieser Ionophor mit Kationen, beziehungsweise Anionen,

beziehungsweise elektrisch neutralen ionogenen Spezien der Probelösung in Berührung kommt oder

B) zwei unterschiedliche kationselektive lipophile Ionophore in Kombination mit einem negativ geladenen Liganden und/oder negativ geladenen Stellen des aus Polymermaterial aufgebauten Trägermaterials enthalten, wobei diese negativen Ladungen durch Kationen elektrisch neutralisiert sind und/oder in Kombination mit mindestens einem Kationenaustauscher enthalten, wobei mindestens einer der beiden kationselektiven lipophilen Ionophore eine chromophore Gruppe aufweist, die in der Lage ist, die optischen Eigenschaften des kationselektiven lipophilen Ionophors zu ändern, sobald dieser mit Kationen oder elektrisch neutralen ionogenen Spezien der Probelösung in Berührung kommt, oder

C) mindestens einen anionselektiven Ionophor in Kombination mit mindestens einem positiv geladenen Liganden und/oder positiv geladenen Stellen des Trägermaterials enthält, wobei diese positiven Ladungen mit Anionen elektrisch neutralisiert sind, und/oder in Kombination mit mindestens einem Anionenaustauscher enthält, wobei mindestens eine dieser Komponenten eine chromophore Gruppe aufweist, die in der Lage ist. die optischen Eigenschaften der Komponente zu ändern, sobald die Testvorrichtung mit Anionen, beziehungsweise elektrisch neutralen ionogenen Spezien der Probelösung in Berührung kommt.

Vorzugsweise sind bei der im Abschnitt B) beschriebenen Ausführungsart die Ladungen von negativ geladenen Liganden, beziehungsweise die Ladungen von negativ geladenen Stellen des Trägermaterials durch andere Kationen als Protonen elektrisch neutralisiert. Es kann nämlich dadurch eine allfällige Störung der Testergebnisse durch eine Aenderung des pH-Wertes der Probelösung vermieden werden.

Vorzugsweise ist der in der erfindungsgemässen Testvorrichtung enthaltene lipophile Ionophor, der eine chromophore Gruppe aufweist, beziehungsweise die in ihr enthaltene Komponente, die eine chromophore Gruppe aufweist, eine solche, die in der Lage ist, eine Fluoreszenz auszubilden oder eine Fluoreszenzlöschung zu ergeben oder eine Veränderung der Lichtabsorption im ultravioletten Bereich, im sichtbaren Bereich oder im infraroten Bereich hervorzurufen, sobald der entsprechende Ionophor, beziehungsweise die entsprechende Komponente mit Kationen, Anionen oder elektrisch neutralen ionogenen Spezien der Probelösung in Berührung kommt.

Ein weiterer Gegenstand der vorliegenden Erfindung ist ein Verfahren zur optischen Bestimmung der Anwesenheit, beziehungsweise Konzentration, beziehungsweise Aktivität von Kationen, Anionen oder elektrisch neutralen ionogenen Spezien in einer im wesentlichen wässrigen Probelösung, wobei dieses Verfahren dadurch gekennzeichnet ist, dass man die wässrige Probelösung mit einer erfindungsgemässen Testvorrichtung in Berührung bringt, wobei entweder durch den Kontakt der Testvorrichtung mit der im wesentlichen wässrigen Probelösung ein simultaner Uebergang von Anionen und Kationen aus der wässrigen Probelösung in das Polymermaterial der Testvorrichtung und/oder ein Austausch von Kationen, beziehungsweise Anionen der Probelösung gegen in dem Polymermaterial der Testvorrichtung enthaltene Kationen, beziehungsweise Anionen, beziehungsweise ein Austausch elektrisch neutraler ionogener Spezien der wässrigen Probelösung gegen elektrisch neutrale ionogene Spezien des Polymermateriales der Testvorrichtung erfolgt, und wobei entweder

a) durch die Anwesenheit des zu bestimmenden Kations, eines mit diesem in Konkurrenz stehenden Kations oder eines Anions, das mit dem zu bestimmenden Kation formal ein Salz bildet, eine Fluorszenz ausgebildet, eine Fluoreszenz gelöscht oder eine Veränderung der Lichtabsorption im ultravioletten Bereich, im sichtbaren Bereich oder im infraroten Bereich hervorgerufen wird oder

b) wobei durch die Anwesenheit des in der Probelösung zu bestimmenden Anions, eines mit diesem konkurrierenden Anions oder eines Kations, welches mit dem zu bestimmenden Anion formal ein Salz bildet, eine Fluoreszenz ausgebildet oder eine Fluoreszenz gelöscht oder eine Veränderung der Lichtabsorption im ultravioletten Bereich, im sichtbaren Bereich oder im infraroten Bereich hervorgerufen wird oder

c) dass durch die Berührung der Testvorrichtung mit einer in der Probelösung enthaltenen elektrisch neutralen ionogenen Spezies, beispielsweise einer Säure, einer Base, einem Ionenassoziat oder einem Zwitterion, eine Fluoreszenz ausgebildet oder eine Fluoreszenz gelöscht oder eine Veränderung der Lichtabsorption im ultravioletten Bereich, im sichtbaren Bereich oder im infraroten Bereich hervorgerufen wird.

Wenn man das erfindungsgemässe Testverfahren mit einer im Abschnitt A) definierten erfindungsgemässen Testvorrichtung durchführt, welche in dem aus Polymermaterial bestehenden Trägermaterlal mindestens einen kationselektiven lipophilen Ionophor und ausserdem mindestens einen anionselektiven lipophilen Ionophor enthält, wobei mindestens einer dieser Ionophore eine chromophore Gruppe aufweist, welche die optischen Eigenschaften des Ionophor ändert, sobald dieser mit dem zu bestimmenden Kation, einem Anion, das mit diesem formal ein Salz bildet, dem zu bestimmenden Anion oder einem Kation, das mit diesem formal ein Salz bildet, beziehungsweise einer zu bestimmenden elektrisch neutralen ionogenen Spezies in Berührung kommt, dann wird durch die Anwesenheit des mindestens einen kationselektiven lipophilen Ionophors und die gleichzeitige Anwesenheit des mindestens einen anionselektiven lipophilen Ionophors eine simultane Coextraktion von Kationen mit der äquivalenten Menge der mit diesen

formal ein Salz bildenden Anionen erreicht oder es wird dadurch die Extraktion einer elektrisch neutralen ionogenen Spezies aus der wässrigen Probelösung in das Polymermaterial der Testvorrichtung durchgeführt. Dadurch, dass in diesem Falle eine simultane Coextraktion äquivalenter Mengen von Kationen und Anionen erfolgt, ist die elektrische Neutralität an der Oberfläche des Polymermaterials gewährleistet, das den kationselektiven lipophilen Ionophor und den anionselektiven lipophilen Ionophor enthält, und diese elektrische Neutralität ist auch im Inneren des Polymermaterials gewährleistet. Ferner wird durch diese simultane Coextraktion von Kationen und äquivalenten Mengen der mit diesen formal ein Salz bildenden Anionen aus der im wesentlichen wässrigen Probelösung in das Polymermaterial gewährleistet, dass der Anteil des kationselektiven lipophilen Ionophors, der mit dem Kation der Probelösung den entsprechenden Kationenkomplex bildet, im Vergleich zu dem nicht komplexierten kationselektiven lipophilen Ionophor, der in dem Polymermaterial der Testvorrichtung enthalten ist, äquivalent mit der Konzentration des entsprechenden Kations in der wässrigen Probelösung ansteigt und gleichzeitig ebenfalls der entsprechende Anteil des anionselektiven lipophilen Ionophors, der in dem Polymermaterial der Testvorrichtung mit dem Anion der Probelösung den Komplex bildet, im Vergleich zu dem in dem Polymermaterial enthaltenen Anteil des freien anionselektiven lipophilen Ionophors, proportional mit dem Gehalt der Probelösung an dem entsprechenden Anion ist.

Wenn also in der entsprechenden Testvorrichtung der in ihr enthaltene kationselektive lipophile Ionophor derjenige Ionophor ist, der eine chromophore Gruppe aufweist, dann kann diese Testvorrichtung dazu verwendet werden, um die Konzentration, beziehungsweise Aktivität der Probelösung an dem zu bestimmenden Kation festzustellen. Wenn die entsprechende Testvorrichtung mit der wässrigen Probelösung in Berührung gebracht wird, dann erfolgt eine simultane Coextraktion des zu bestimmenden Kations mit dem Anion, das mit diesem formal ein Salz bildet, aus der Probelösung in das den kationselektiven lipophilen Ionophor und den anionselektiven lipophilen Ionophor enthaltende Polymermaterial und der Anteil des kationselektiven lipophilen Ionophors, der mit dem Kation in Wechselwirkung tritt, im Vergleich zu dem Anteil des unkomplexiert verbleibenden kationselektiven lipophilen Ionophors steigt äquivalent mit der Konzentration, beziehungsweise Aktivität des entsprechenden Kations in der Probelösung. Dementsprechend ist auch die Aenderung der optischen Eigenschaften dieses kationselektiven lipophilen Ionophors äquivalent zu der Konzentration, beziehungsweise Aktivität der zu bestimmenden Kationen in der Probelösung.

Durch die erwähnte simultane Coextraktion von Kationen und der mit diesen formal ein Salz bildenden Anionen aus der Probelösung in das Polymermaterial der Testvorrichtung, steigt bei einer höheren Konzentration an dem Kation in der Probelösung auch die äquivalente Menge des Anions, das aus der Probelösung mit diesem Kation coextrahiert wird. Wenn nun in der beschriebenen Ausführungsart der in dem Polymermaterial der Testvorrichtung vorhandene kationselektive lipophile Ionophor derjenige ist, der die chromophore Gruppe aufweist, während der in dem Polymermaterial enthaltene anionselektive lipophile Ionophor keine chromophore Gruppe trägt, dann eignet sich die entsprechende Vorrichtung auch zur Bestimmung der Konzentration, beziehungsweise Aktivität des Anions, das mit dem Kation der Probelösung formal ein Salz bildet. Bei der Anwesenheit höherer Konzentrationen an dem zu bestimmenden Anion in der wässrigen Probelösung werden gleichzeitig auch höhere Konzentrationen des Kations, das mit diesem ein Salz bildet, aus der Probelösung in das Polymermaterial der Testvorrichtung coextrahiert und diese höheren Konzentrationen des Kations führen grössere Mengen des kationselektiven lipophilen Ionophors, der eine chromophore Gruppe aufweist, in den entsprechenden Komplex über, sodass durch eine stärkere Aenderung der optischen Eigenschaften des kationselektiven lipophilen Ionophors die Anwesenheit der korrespondierenden höheren Menge der zu bestimmenden Anionen in der Probelösung nachgewiesen wird.

Aus den obigen Erläuterungen ist ersichtlich, dass eine erfindungsgemässe Testvorrichtung gemäss Abschnitt A), die mindestens einen kationselektiven lipophilen Ionophor und ausserdem mindestens einen anionselektiven lipophilen Ionophor enthält, wobei der anionselektive lipophile Ionophor derjenige ist, der eine chromophore Gruppe aufweist, die in der Lage ist, ihre optischen Eigenschaften zu ändern, sobald der Ionophor mit dem Anion in Berührung kommt, für das dieser anionenselektive lipophile Ionophor empfindlich ist, entweder zur Bestimmung der Konzentration, beziehungsweise Aktivität des entsprechenden Anions in der Probelösung herangezogen werden kann oder auch zur Bestimmung der Konzentration, beziehungsweise Aktivität desjenigen Kations, das mit dem fraglichen Anion formal ein Salz bildet.

Die im Abschnitt A) definierte erfindungsgemässe Testvorrichtung ist auch geeignet, um die Konzentration, beziehungsweise Aktivität einer elektrisch neutralen ionogenen Spezies in der Probelösung zu bestimmen, weil durch die Anwesenheit des mindestens einen kationselektiven lipophilen Ionophor und die Anwesenheit des mindestens einen anionselektiven lipophilen Ionophors mit steigender Aktivität, beziehungsweise Konzentration der elektrisch neutralen ionogenen Spezies in der Probelösung, steigende Anteile des kationselektiven lipophilen Ionophors und anionselektiven lipophilen Ionophors in den entsprechenden Komplex übergeführt werden und damit unabhängig davon, ob jetzt der kationselektive lipophile Ionophor derjenige ist, der seine optischen Eigenschaften bei der Komplexbildung ändert, oder der anionselektive lipophile Ionophor derjenige ist, der seine optischen Eigenschaften bei der Komplexbildung ändert, die Aenderung der optischen Eigenschaften der Testvorrichtung mit der Konzentration, beziehungsweise Aktivität der elektrisch neutralen ionogenen Spezies in der Probelösung äquivalent ist.

Beispiele für elektrisch neutrale ionogene Spezien, deren Aktivität, beziehungsweise Konzentration mit diesem

Testverfahren bestimmt werden kann, sind Säuren (es stellt sich das Gleichgewicht zwischen der in $H_3O^+$ Ionen und negative geladene Anionen dissoziierte Säure mit der entsprechenden undissoziierten Säure ein) oder die elektrisch neutrale ionogene Spezies kann eine Base sein. Des weiteren kann die elektrisch neutrale ionogene Spezies auch ein Zwitterion sein.

Als Beispiele für Zwitterionen, deren Aktivität, beziehungsweise Konzentration durch dieses Testverfahren bestimmbar ist, seien Betaine, beziehungsweise in der zwitterionischen Form vorliegende Aminosäuren erwähnt.

Als Beispiele für elektrisch neutrale ionogene Spezien, die ein Ionenassoziat darstellen, seien Ionenpaare oder elektrisch neutrale Assoziate aus mehreren Ionen erwähnt. So wurde beispielsweise bei wässrigen Lösungen von Kupfersulfat festgestellt, dass darin Assoziate aus einem Kupferion und einem Sulfatanion, die meistens noch über Wassermoleküle miteinander verbunden sind, vorliegen können, wobei derartige Assoziate elektrisch neutral sind. Ensprechende Ionenassoziate können auch bei konzentrierten Lösungen von anderen Metallsulfaten, Metallnitraten oder Metallcarbonaten vorliegen.

In den erfindungsgemässen Testvorrichtungen ist das Trägermaterial ein im wesentlichen nicht poroses Polymermaterial, das in der zu testenden, im wesentlichen wässrigen Probelösung praktisch unlöslich, allenfalls leicht quellbar ist. die Polymermaterialien sind Homopolymerisate oder Copolymerisate, die nur geringe Anteile an hydrophilen Gruppen aufweisen oder frei von hydrophilen Gruppen sind, wie zum Beispiel Polyvinylhalogenidhomopolymerisate, Polyvinylhalogenidcopolymerisate, Polyvinylidenhalogenidhomopolymerisate und Polyvinylidenhalogenidcopolymerisate. Als spezielles Beispiel seien Polyvinylchloridhomopolymerisate und Copolymerisate aus Polyvinylchlorid mit geringeren Anteilen an Polyvinylalkohol oder Copolymerisate aus Polyvinylchlorid mit geringeren Anteilen an Polyvinylacetat und Polyvinylalkohol erwähnt.

Gegebenenfalls können die Trägermaterialien als weitere Komponente auch noch Weichmacher enthalten, beispielsweise Weichmacher auf Basis von Dicarbonsäurediestern oder auf Basis von Tetracarbonsäuretetraestern, in denen der esterbildende Alkohol im allgemeinen ein Alkanol mit mindestens vier Kohlenstoffatomen ist.

Aus den obigen Erläuterungen sieht man also, dass die erfindungsgemässen Testvorrichtungen eine organische Phase darstellen. Diese wird durch das im wesentlichen lipophile das Trägermaterial darstellende Polymer gebildet, das die weiteren Komponenten enthält, nämlich entweder

mindestens einen kationselektiven lipophilen Ionophor, kombiniert mit mindestens einem anionselektiven lipophilen Ionophor und/oder mit mindestens einem negativ geladenen Liganden und/oder negativ geladenen Stellen des Trägermaterials enthält, wobei die negativen Ladungen mit Kationen elektrisch neutralisiert sind, und/oder in Kombination mit mindestens einem Kationenaustauscher enthält, oder das
mindestens einen lipophilen anionselektiven Ionophor in Kombination mit mindestens einem kationselektiven lipophilen Ionophor und/oder in Kombination mit mindestens einem positiv geladenen Liganden und/oder positiv geladenen Stellen des Trägermaterials enthält, wobei diese positiven Ladungen mit Anionen elektrisch neutralisiert sind und/oder in Kombination mit mindestens einem Anionenaustauscher. Diese organische Phase steht bei der Verwendung der erfindungsgemässen Testvorrichtung im Gleichgewicht mit der im wesentlichen wässrigen Probelösung.

Wenn man den Test mit einer im Abschnitt B) beschriebenen erfindungsgemässen Testvorrichtung durchführt, die zwei unteischiedliche kationselektive lipophile Ionophore in Kombination mit einem negativ geladenen Liganden und/oder negativ geladenen Stellen des aus Polymermaterial bestehenden Trägermaterials und/oder mindestens einem Kationenaustauscher enthält, wobei diese negativen Ladungen durch Kationen elektrisch neutralisiert sind, wobei mindestens einer der beiden Kationselektiven lipophilen Ionophore eine chromophore Gruppe aufweist, die in der Lage ist, die optischen Eigenschaften des Kationselektiven lipophilen Ionophors zu ändern, sobald diese mit Kationen oder elektrisch neutralen ionogenen Spezien der Probelösung in Berührung kommt, dann wird durch die Anwesenheit des kationselektiven lipophilen Ionophor und die Anwesenheit der negativ geladenen Liganden, beziehungsweise der negativ geladenen Stellen des Trägermaterials, die jeweils aus Gründen der Elektroneutralität mit Kationen elektrisch neutralisiert sein müssen, beziehungsweise durch die Anwesenheit des Kationenaustauschers eine simultane Coextraktion von Kationen und Anionen der Probelösung oder eine Extraktion einer elektrisch neutralen ionogenen Spezies aus der Probelösung in das Polymermaterial der Testvorrichtung hervorgerufen und/oder

ein simultaner Ionenaustausch von Kationen der wässrigen Probelösung mit Kationen des Polymermateriales der Testvorrichtung hervorgerufen.

Auch in diesem Falle ist die Menge an dem kationselektiven lipophilen Ionophor, die bei einer Berührung mit der wässrigen Probelösung in den entsprechenden Kationkomplex übergeführt wird, äquivalent zu der Konzentration, beziehungsweise Aktivität des entsprechenden Kations, gegenüber dem der lipophile Ionophor selektiv ist, in der wässrigen Probelösung.

Bei Anwesenheit höherer Konzentrationen, beziehungsweise Aktivitäten an dem fraglichen Kation in der wässrigen Probelösung, treten auch höhere Konzentrationen an dem fraglichen Kation in die organische Phase des Polymermaterials ein und es bilden sich dort höhere Konzentrationen an dem Komplex zwischen dem fraglichen Kation und dem kationselektiven lipophilen Ionophor im Vergleich zu dem entsprechenden unkomplexierten kationselektiven lipophilen Ionophor. Aufgrund der Elektroneutralität muss jedoch gleichzeitig eine Coextraktion äquivalenter Mengen der formal salzbildenden Anionen aus der wässrigen Probelösung in das aus Polymermaterial aufgebaute Trägermaterial erfolgen und/oder es muss ein simultaner Kationenaustausch von Kationen, die im Polymermaterial enthalten sind, gegen Kationen der wässrigen Probelösung stattfinden. Die entsprechenden austauschbaren Kationen liegen im Polymermaterial in gebundener Form vor und zwar sind sie entweder an negativ geladene Liganden gebunden oder sie sind an negativ geladene Stellen des Polymermateriales gebunden und/oder sie sind in einem Kationenaustauscher anwesend, der sich in dem Polymermaterial befindet.

Aufgrund der lipophilen Eigenschaften des Polymermateriales ist es vorteilhaft, wenn die austauschbaren Kationen an einem negativ geladenen Liganden gebunden sind, der relativ stark lipophile Eigenschaften aufweist. Beispiele für derartige lipophile negativ geladene Liganden sind organische Borate, wie beispielsweise das Kalium-tetrakis(phenyl) borat oder das Kalium-tetrakis(p-chlorphenyl)borat.

Wenn man den Test mit einer im Abschnitt C) beschriebenen Testvorrichtung durchführt, die mindestens einen anionselektiven Ionophor in Kombination mit mindestens einem positiv geladenen Liganden und/oder positiv geladenen Stellen des aus Polymermaterial bestehenden Trägermaterials und/oder mindestens einem Anionenaustauscher enthält, wobei mindestens eine dieser Komponenten eine chromophore Gruppe aufweist, durch welche die optischen Eigenschaften dieser Komponente geändert werden, sobald diese mit Anionen, beziehungsweise elektrisch neutralen ionogenen Spezien der Probelösung in Berührung kommt, dann erfolgt durch die Anwesenheit des anionselektiven Ionophors in Kombination mit mindestens einem positiv geladenen Liganden, der aufgrund der Elektroneutralität von einem Anion abgesättigt ist und/oder in Kombination mit positiv geladenen Stellen des Trägermaterials, die aufgrund der Elektroneutralität mit Anionen abgesättigt sind und/oder aufgrund der Anwesenheit eines Anionenaustauschers eine simultane Coextraktion von Kationen und Anionen aus der Probelösung oder eine Extraktion einer elektrisch neutralen ionogenen Spezies aus der Probelösung in das Polymermaterial der Testvorrichtung und/oder es erfolgt ein simultaner Ionenaustausch von Anionen der wässrigen Probelösung gegen Anionen des Polymermateriales der Testvorrichtung.

Ein Beispiel für einen positiv geladenen Liganden, der mit einem entsprechenden Anion abgesättigt ist, ist ein quaternäres Ammoniumsalz. Da als Trägermaterial im wesentlichen lipophile Eigenschaften aufweisende Polymermaterialien eingesetzt werden, sind solche quaternäre Ammoniumsalze bevorzugt, die selbst stärker lipophile Eigenschaften besitzen, damit die entsprechende Löslichkeit in dem Polymermaterial gewährleistet ist. Bevorzugte quaternäre Ammoniumsalze besitzen also mindestens eine lipophile längerkettige aliphatische Gruppe, mindestens einen grösseren cycloaliphatischen Rest und/oder mindestens einen gegebenenfalls substituierten aromatischen Rest. Als spezielles Beispiel sei Methyltridodecylammoniumchlorid genannt.

Wenn eine Testvorrichtung, die einen an ionselektiven lipophilen Ionophor und ein lipophile Eigenschaften aufweisendes quaternäres Ammoniumsalz in dem Polymermaterial enthält, mit einer wässrigen Probelösung in Berührung gebracht wird, die das Anion enthält, gegenüber dem der lipophile Ionophor selektiv ist, dann tritt dieses Anion aus der wässrigen Probelösung in das Polymermaterial der Testvorrichtung ein und bildet mit dem Ionophor den Komplex. Gleichzeitig wird eine äquivalente Menge an Anionen des quaternären Ammoniumsalzes in die Probelösung abgegeben. Im Falle des oben erwähnten Methyltridodecylammoniumchlorids tritt also eine äquivalente Menge an Chloridionen aus dem Polymermaterial in die wässrige Probelösung aus. Wenn ferner der anionselektive lipophile Ionophor ein gegenüber Carbonatanion selektiver Ionophor ist, dann lässt sich dieser Ionenaustausch durch das folgende Rekationsschema veranschaulichen:

$$CO_3^{2-}(aq) + 2\,Cl^-(org) \rightleftarrows CO_3^{2-}(compl.,org) + 2\,Cl^-(aq)$$

Wenn eine Testvorrichtung mit ionenselektiven Ionophoren für Bicarbonatanionen selektiv ist und als positiv geladener Ligand mit neutralisierendem Anion Methyltridodecylammoniumchlorid eingesetzt wird, dann verläuft der Austausch der Bicarbonatanionen der wässrigen Probelösung gegen die Chloridanionen, die in dem Polymermaterial der Testvorrichtung gebunden sind, nach dem folgenden Reaktionsschema:

$$HCO_3^-(aq) + Cl^-(org) \rightleftarrows H^+(compl.,org) + CO_3^{2-}(compl.,org) + Cl^-(aq)$$

Im erstgenannten Fall ist also das Ausmass der Komplexierung der Carbonatanionen $CO_3^{2-}$ in der organischen Phase des Polymermateriales äquivalent mit der Aktivität, beziehungsweise Konzentration der Carbonatanionen in der wässrigen Phase, und zwar deshalb, weil bei dem Uebergang eines Aequivalents an Carbonatanionen aus der wässrigen Phase in die organische Phase des Polymermateriales zwei Aequivalente an Chloridanionen von der organischen Phase in die wässrige Phase abgegeben werden. Es ist also das Ausmass der durch die Komplexbildung mit dem anionselektiven Ionophor hervorgerufenen Aenderung der optischen Eigenschaften in der Testvorrichtung proportional zu der Konzentration, beziehungsweise Aktivität des zu bestimmenden Anions in der Probelösung.

Dieser zuerst genannte Fall stellt also ein Beispiel für einen positiv geladenen Liganden dar, dessen positive Ladungen mit Anionen elektrisch neutralisiert sind, wobei bei der Durchführung des Tests ein Anionenaustausch zwischen den zu bestimmenden Anionen der wässrigen Probelösung und den diese positiven Ladungen neutralisierenden Anionen des Polymermateriales der Testvorrichtung stattfindet.

Im zweiten hier dargestellten Fall treten beim Kontakt der Testvorrichtung mit der wässrigen Probelösung die zu bestimmenden Bicarbonatanionen $HCO_3^-$ aus der wässrigen Probelösung in die organische Phase des Polymermaterials über und eine äquivalente Menge an Chloridanionen geht von der organischen Phase des Polymermaterials in die wässrige Probelösung über. Auch hier ist, wie aus der obigen Reaktionsgleichung ersichtlich ist, das Ausmass der Bildung des Carbonatkomplexes mit dem anionselektiven Ionophor, das in dem Polymermaterial der Testvorrichtung erfolgt, äquivalent zu der Konzentration, beziehungsweise Aktivität der Bicarbonatanionen in der wässrigen Probelösung. Da aber in der organischen Phase des Polymermateriales nicht das Bicarbonatanion $HCO_3^-$ selbst mit dem anionselektiven Ionophor komplexiert wird, sondern dieses in der organischen Phase in Protonen und Carbonatanionen $CO_3^{2-}$ dissoziiert und diese beiden Ionenarten dann durch die entsprechenden selektiven Ionophore komplexiert werden, handelt es sich bei diesem zweiten Modellfall sozusagen um eine Uebergangsform zwischen einem reinen System einer simultanen Coextraktion von Kationen und Anionen aus der Probelösung in das Polymermaterial der Testvorrichtung und einem reinen Ionenaustausch von Anionen der Probelösung gegen Anionen des Polymermateriales der Testvorrichtung.

Beispiele für anionselektive lipophile Ionophore, die in den erfindungsgemässen Testvorrichtungen verwendet werden können, sind beliebige in der Literatur beschriebene anionselektive Ionophore, beispielsweise diejenigen, welche in der nicht zum Stande der Technik gehörenden europäischen Patentveröffentlichung Nr. 0 281 829 der Firma Willi Möller beschrieben sind. Diese sind in der Lage, mit Anionen verschiedener anorganischer Oxasäuren, beispielsweise Sulfationen, Phosphationen oder Carbonationen, oder mit Anionen organischer Oxasäuren, beispielsweise Carbonsäure, in Wechselwirkung zu treten, wobei sich im allgemeinen Komplexe zwischen den dort beschriebenen Ketoverbindungen mit den Oxasäuren ausbilden.

Bevorzugte derartige anionselektive lipophile Ionophore sind Carbonylverbindungen, die eine chromophore Gruppe aufweisen und wobei in diesen Carbonylverbindungen die Carbonylgruppe einen Teil des Chromophoren darstellt oder in Wechselwirkung mit einer fluoreszierenden Gruppe steht. Wenn dann die fraglichen Ketoverbindungen mit einem Anion einer Oxasäure oder einer entsprechenden elektrisch neutralen ionogenen Spezies in Wechselwirkung treten, dann wird eine Aenderung der Absorption im ultravioletten Bereich, im sichtbaren Bereich oder im infraroten Bereich hervorgerufen, oder es wird durch das Inwechselwirkungtreten der Ketoverbindung mit einem Anion einer Oxasäure, beziehungsweise einer entsprechenden elektrisch neutralen ionogenen Spezies eine Fluoreszenz hervorgerufen oder eine Fluoreszenz gelöscht.

Beispiele für entsprechende anionselektive Ionophore sind Ketoverbindungen der Formel I oder III

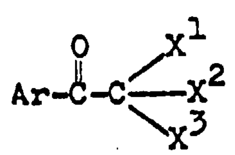

. I

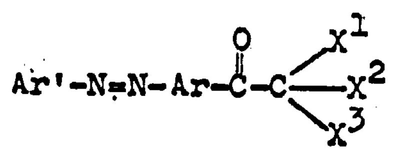

III

wobei in den Verbindungen der Formel I, beziehungsweise III die Reste

Ar und Ar' unabhängig voneinander substituierte oder unsubstituierte, ein- oder mehrkernige aromatische, beziehungsweise substituierte oder unsubstituierte, ein- oder mehrkernige heterocyclische Reste aromatischen Charakters bedeuten und

$X^1$, $X^2$ und $X^3$ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer

der Substituenten $X^1$, $X^2$ und $X^3$ ein stark elektronenanziehender Substituent, ausgewählt aus der Gruppe Fluoratome, Chloratome, Bromatome oder Nitrogruppen ist, und

wobei dann, wenn die Ketoverbindung der Formel I, beziehungsweise III, mit Anionen einer Oxasäure oder entsprechenden elektrisch neutralen ionogenen Spezien in Wechselwirkung tritt, eine Aenderung der Absorption im ultravioletten Bereich, im sichtbaren Bereich oder im Infrarotbereich auftritt.

In speziell bevorzugten Ketoverbindungen der Formel I oder III stellen sämtliche Reste $X^1$, $X^2$ und $X^3$ Fluoratome dar.

Aufgrund der Elektroneutralität der erfindungsgemässen Testvorrichtungen ist es ferner möglich, in den erfindungsgemässen Testvorrichtungen zwei unterschiedliche kationselektive lipophile lonophore einzusetzen, die gegenüber unterschiedlichen Arten von Kationen selektiv sind, wobei der eine der beiden kationselektiven lipophilen lonophoren seine optischen Eigenschaften ändert, sobald er mit denjenigen Kationen der Probelösung in Berührung kommt, für die dieser lonophor selektiv ist, während der andere der beiden unterschiedlichen kationselektiven lipophilen lonophore keine Aenderung der optischen Eigenschaften zeigt, wenn er mit dem zu bestimmenden Kation in Berührung kommt. Auch in diesem Falle muss das Polymermaterial der Testvorrichtung zusätzlich zu den beiden kationselektiven lipophilen lonophoren noch einen negativ geladenen Liganden und/oder negativ geladene Stellen des Trägermaterials enthalten, wobei diese negativen Ladungen durch Kationen elektrisch neutralisiert sind und/oder zusätzlich einen Kationaustauscher enthalten.

Wenn bei dieser bevorzugten Auführungsart der erfindungsgemässen Testvorrichtungen gemäss der Variante B der erste kationselektive lipophile lonophor, der für das zu bestimmende Kation selektiv ist, seine optischen Eigenschaften nicht ändert. wenn er mit dem zu bestimmenden Kation in Berührung kommt, ledoch der zweite kationselektive lipophile lonophor seine optischen Eigenschaften ändert, wenn er mit einem anderen Kation in Berührung kommt, das ebenfalls in der Probelosung enthalten ist, wobei jedoch die Konzentration an diesem anderen Kation in der Probelösung immer konstant bleibt, dann kann diese Testvorrichtung zur optischen Bestimmung des zu bestimmenden Kations herangezogen werden, weil äquivalent mit der Komplexierung des ersten kationselektiven lipophilen lonophors durch Uebertritt des zu bestimmenden Kations aus der Probelösung in das Polymermaterial der Testvorrichtung ein Kation des Kationenkomplexes des zweiten kationselektiven lipophilen lonophors aus dem Polymermaterial in die wässrige Probelösung übertritt und dadurch die Aenderung der optischen Eigenschaften des zweiten kationselektiven lipophilen lonophors feststellbar ist.

Analog sind die Verhältnisse bei erfindungsgemässen Testvorrichtungen, die zwei unterschiedliche anionselektive lipophile lonophore aufweisen, wobei der für das zu bestimmende Anion selektive lipophile lonophor keine chromophore Gruppe aufweist, und nur der für das zweite Anion selektive lonophor, eine chromophore Gruppe aufweist, welche die optischen Eigenschaften dieses zweiten anionselektiven lonophors ändert, sobald dieser mit dem zweiten Anion in Berührung kommt. Auch in diesem Falle muss das Polymermaterial der Testvorrichtung zusätzlich zu den beiden anionselektiven lipophilen lonophoren noch mindestens einen positiv geladenen Liganden und/oder positiv geladene Stellen des Polymermaterials enthalten, wobei diese positiven Ladungen mit Anionen elektrisch neutralisiert sind und/ oder zusätzlich mindestens einen Anionenaustauscher enthalten. Auch hier muss gewährleistet sein, dass die Konzentration des zweiten Anions, für das der zweite lipophile anionselektive lonophor selektiv ist. in der Probelösung jeweils konstant bleibt, damit aufgrund der Elektroneutralität durch die Aequivalenz des Eintrittes des ersten zu bestimmenden Anions aus der Probelösung in das Polymermaterial der Testvorrichtung und des zweiten nicht zu bestimmenden Anions aus dem Polymermaterial in die Probelösung die Konzentration des ersten Anions durch eine Aenderung der optischen Eigenschaften des zweiten anionselektiven lipophilen lonophors feststellbar ist.

Eine bevorzugte Ausführungsart der Erfindung betrifft dementsprechend solche Testvorrichtungen, die C) zwei unterschiedliche anionselektive lipophile lonophore in Kombination mit mindestens einem positiv geladenen Liganden und/oder positiv geladenen Stellen des aus Polymermaterial bestehenden Trägermaterials enthalten, wobei diese positiven Ladungen mit Anionen elektrisch neutralisiert sind und/oder in Kombination mit mindestens einem Anionenaustauscher enthalten, wobei mindestens einer der beiden anionselektiven lipophilen lonophore eine chromophore Gruppe aufweist, die in der Lage ist, die optischen Eigenschaften dieses lonophors zu ändern, sobald dieser mit Anionen oder elektrisch neutralen ionogenen Spezien der Probelösung in Berührung kommt.

Wenn bei der Durchführung des Testverfahrens die Probelösung mit einer dieser bevorzugten Testvorrichtungen, die oben unter Abschnitt B) beschrieben sind, in Berührung kommt, dann geht das zu bestimmende Kation, beziehungsweise die zu bestimmende elektrisch neutrale ionogene Spezies aus der im wesentlichen wässrigen Probelösung in das Polymermaterial der Testvorrichtung über und wird dort an den für dieses Kation, beziehungsweise elektrisch neutrale ionogene Spezies, selektiven lipophilen lonophor gebunden und gleichzeitig wird eine äquivalente Menge eines unterschiedlichen Kations, das in dem Polymermaterial an den zweiten kationselektiven lipophilen lonophor gebunden ist, beziehungsweise eine unterschiedliche elektrisch neutrale ionogene Spezies aus diesem Polymermaterial in die wässrige Probelösung abgegeben. Wenn der kationselektive lonophor, der für das zu bestimmende Kation, beziehungsweise die elektrisch neutrale ionogene Spezies selektiv ist, eine chromophore Gruppe aufweist, dann ist

die Konzentration des zu bestimmenden Kations, beziehungsweise der elektrisch neutralen ionogenen Spezies. in der Probelösung durch eine äquivalente Veränderung der optischen Eigenschaften dieses kationselektiven lipophilen Ionophors feststellbar. Wenn der zweite kationselektive lipophile Ionophor derjenige ist, der eine chromophore Gruppe aufweist, dann ist die Konzentration des zu bestimmenden Kations, beziehungsweise der elektrisch neutralen ionogenen Spezies. in der Probelösung durch eine Aenderung der optischen Eigenschaften dieses zweiten kationselektiven lipophilen Ionophors infolge der Freisetzung der anderen Kationenart an die Probelösung feststellbar.

Analog sind die Verhältnisse, wenn man die Probelösung mit einer zwei unterschiedliche anionselektive lipophile Ionophore enthaltenden bevorzugten Testvorrichtung in Berührung bringt, wie sie oben im Abschnitt C) beschrieben ist. In diesem Fall, wird bei dem Kontakt der Probelösung mit dem Polymermaterial der Testvorrichtung das zu bestimmende Anion, beziehungsweise die entsprechende elektrisch neutrale ionogene Spezies aus der Probelösung in das Polymermaterial der Testvorrichtung befördert und an den für diese Komponente selektiven lipophilen Ionophoren gebunden und gleichzeitig wird eine äquivalente Menge eines Anions, beziehungsweise einer elektrisch neutralen ionogenen Spezies von dem zweiten lipophilen Ionophor an die wässrige Probelösung abgegeben. Wenn der für das zu bestimmende Anion selektive lipophile Ionophor eine chrompohore Gruppe aufweist, ist die Konzentration, beziehungsweise Aktivität des zu bestimmenden Anions, beziehungsweise der elektrisch neutralen ionogenen Spezies der Probelösung durch die Aenderung der optischen Eigenschaften dieses anionselektiven lipophilen Ionophors feststellbar. Wenn jedoch der für das zweite Anion selektive lipophile Ionophor eine chromophore Gruppe aufweist, dann ist die Konzentration, beziehungsweise Aktivität des zu bestimmenden Anions, beziehungsweise der entsprechenden elektrisch neutralen ionogenen Spezies, durch die Aenderung der optischen Eigenschaften dieses Ionophors aufgrund der Freigabe des zweiten Anions an die Probelösung feststellbar.

Speziell bevorzugt sind von den erfindungsgemässen Testvorrichtungen der Variante B) solche, die einen kationselektiven lipophilen Ionophor aufweisen, der gegenüber dem zu bestimmenden Kation selektiv ist und ferner einen zweiten kationselektiven lipophilen Ionophor, der gegenüber Proton selektiv ist und der in der protonierten Form andere optischen Eigenschaften aufweist als in der nicht protonierten Form. Bei Einhaltung eines konstanten pH-Wertes der Probelösung, beispielsweise bei Einstellung des pH-Wertes der Probelösung auf einen konstanten Wert durch Verwendung eines Puffers, kann somit die Konzentration des zu bestimmenden Kations durch die Aenderung der optischen Eigenschaften des gegenüber Protonen empfindlichen zweiten kationselektiven lipophilen Ionophors festgestellt werden.

Besonders vorteilhaft ist es, dass in diesem Fall ein einziger gegenüber Protonen empfindlicher Ionophor, der eine chromophore Gruppe aufweist, welche die optischen Eigenschaften dieses Ionophors ändert, sobald er mit Protonen in Berührung kommt, zur Bestimmung unterschiedlicher Arten an Kationen in Probelösungen eingesetzt werden kann, sofern man diesen gegenüber Protonen selektiven Ionophor in der Testvorrichtung mit einem der üblichen in der Literatur beschriebenen kationselektiven lipophilen Ionophoren kombiniert, die eine Selektivität für die jeweils zu bestimmenden Kationen aufweisen.

Aufgrund dieser Erkenntnise wurden jetzt neue gegenüber Protonen selektive lipophile Ionophore entwickelt, und zwar ausgehend von üblichen in der Literatur beschriebenen Farbindikatoren, die bei Aenderung des pH-Wertes eine Farbveränderung zeigen, indem man diese Farbindikatoren durch Einführung lipophiler Reste so modifizierte, dass sie die erforderlichen lipophilen Eigenschaften aufweisen. Beispiele für derartige lipophile Reste sind langkettige aliphatische Reste, beziehungsweise aromatische Reste, insbesondere solche, die durch langkettige aliphatische Reste substituiert sind.

Speziell bevorzugte neue erfindungsgemässe gegenüber Protonen selektive lipophile Ionophore, die in der mit Protonen komplexierten Form andere optischen Eigenschaften aufweisen als in der freien Form weisen die folgende Formel II

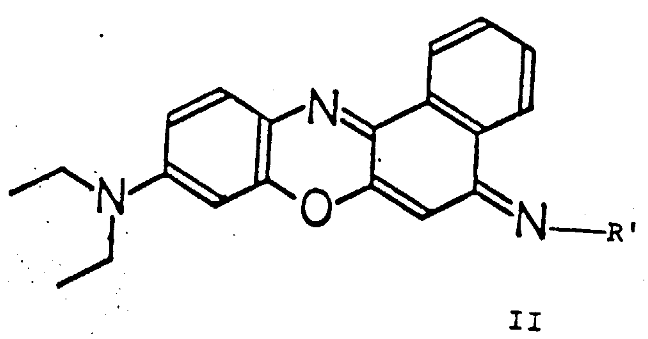

II

auf, wobei in dieser Formel R' ein mindestens 10 Kohlenstoffatome aufweisender aliphatischer Rest ist, der direkt an

das Stickstoffatom oder über eine zweiwertige Gruppe, beispielsweise einen aromatischen Rest, einen cycloaliphatischen Rest, eine Gruppe der Formel -CO- oder -CS- gebunden ist.

Spezielle Beispiele dieser gegenüber Protonen selektiven lipophilen Ionophore der Formel II sind diejenigen, in denen der Rest R' eine Gruppe der Formel

$$-CO-R''$$

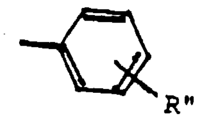

ist, wobei in dieser Formel R" ein geradkettiger Alkylrest mit 10 - 25 Kohlenstoffatomen ist, beispielsweise ein entsprechender Rest mit 15 - 18 Kohlenstoffatomen.

Diese neuen gegenüber Protonen selektiven lipophilen Ionophore sind dementsprechend in Testvorrichtungen der Variante B) mit beliebigen gegenüber speziellen Arten von Kationen selektiven Ionophoren kombinierbar, wobei die Farbveränderungen durch den gegenüber Protonen selektiven Chromoionophor der oben angegebenen Formel II feststellbar ist, während der gegenüber dem zu bestimmenden Kation selektive Ionophor bei Kontakt mit diesem Kation seine Farbe nicht ändert. Typische Beispiele für in Kombination mit der Verbindung der Formel II einsetzbare kationselektive Ionophore sind solche, die gegenüber Alkalimetallen, beispielsweise Natrium, Kalium oder Lithium, sowie gegenüber Erdalkalimetallen, beispielsweise Calcium, Magnesium und Barium, sowie gegenüber Ammoniumionen selektiv sind.

Bei der praktischen Anwendung weisen die erfindungsgemässen Testvorrichtungen, mit denen die Anwesenheit, beziehungsweise Konzentration, beziehungsweise Aktivität von Kationen, Anionen oder elektrisch neutralen ionogenen Spezien durch eine Aenderung einer optischen Eigenschaft feststellbar ist, grosse Vorteile im Vergleich zu entsprechenden Testvorrichtungen auf, bei denen die entsprechende Anwesenheit, Konzentration oder Aktivität mit Hilfe einer ionenselektiven Elektrode bestimmt werden muss.

Entsprechende Testvorrichtungen in Form von Teststreifen aus Polymermaterial oder Ähnlichem Testvorrichtungen müssen lediglich in die im wesentlichen wässrige Probelösung eingetaucht werden. Die Aenderung der optischen Eigenschaften, beispielsweise eine Verfärbung, das Entstehen einer Farbe oder das Auftreten oder Löschen einer Fluoreszenz kann unmittelbar festgestellt werden. Derartige Anwendungen sind in vielen technischen Bereichen, beispielsweise bei der Feststellung einer ionogenen Spezies in einem Produktstrom oder in Abwässern von grosser Bedeutung.

Besonders vorteilhaft sind derartige Testvorrichtungen auch im medizinischen Bereich, weil so eine einfache Bestimmung von Anionen, Kationen oder elektrisch neutralen ionogenen Spezien in unterschiedlichen biologischen Materialien, wie zum Beispiel Körperflüssigkeiten, wie Blutserum, Blutplasma, Gesamtblut oder Harn möglich ist.

Wenn die erfindungsgemässe Testvorrichtung der selektive Bestandteile einer Optode ist, dann kann dieser selektive Teil der Optode in unterschiedliche, schwer zugängliche Stellen eingeführt werden, und von aussen wird dann die entsprechende Ionenaktivität, beziehungsweise Ionenkonzentration durch Beobachtung der Veränderung der optischen Eigenschaften des selektiven Bestandteils feststellbar beispielsweise durch eine entsprechende Uebertragung mit Lichtleitern. Entsprechende Optoden sind in verschiedenen technischen Bereichen und insbesondere in der medizinischen Anwendung von grossem Vorteil, wo sie in schwer zugängliche Bereiche des Körpers, beispielsweise in Magen-Darm-Trakt, Arterien, Venen oder die Fruchtblase einer Schwangeren eingeführt werden, wobei dann von aussen die entsprechende Ionenkonzentration oder Aktivität durch eine Veränderung der optischen Eigenschaften feststellbar ist.

In manchen Fällen ist es sogar möglich, durch die Bestimmung der Ionenkonzentration einen biologischen Vorgang, beispielsweise einen Stoffwechselvorgang von aussen zu beobachten. Die Atmungsaktivität der Haut kann beispielsweise durch Aufbringung eines befeuchteten Teststreifens und die Bestimmung des austretenden und in der wässrigen Phase sich lösenden Kohlendioxides festgestellt werden. Auf diesem Wege ist sogar eine Abschätzung des $CO_2$ Partialdruckes des Blutes möglich, beispielsweise indem man im Zuge eines Geburtsvorganges einen entsprechenden Teststreifen auf die Haut des ungeborenen Kindes aufbringt und dadurch den $CO_2$ Partialdruck des kindlichen Blutes abschätzen kann.

Gemäss einer weiteren Ausführungsart der Erfindung wird das Testverfahren angewandt, um in einer Probelösung die Anwesenheit, beziehungsweise Konzentration, beziehungsweise Aktivität, einer nicht ionogenen Spezies zu bestimmen. In diesem Fall wird die nicht ionogene Spezies durch irgendeine Reaktion, beispielsweise eine Dissoziationsreaktion, eine enzymatisch katalysierte Reaktion oder einen ähnlichen Vorgang, in mindestens ein Kation und/oder Anion und/oder eine elektrisch neutrale ionogene Spezies umgewandelt und die Anwesenheit, beziehungsweise Konzentration, beziehungsweise Aktivität dieser ionogenen Spezies wird dann mit der Testvorrichtung bestimmt.

Ein derartiges Testverfahren ist beispielsweise in der medizinischen Anwendung zur Bestimmung von Harnstoff in biologischem Material, wie zum Beispiel Blutserum oder Gesamtblut anwendbar, wobei der Harnstoff, beispielsweise enzymatisch, in Ammoniumcarbonat übergeführt und dieses dann in der Probelösung bestimmt wird, und zwar entweder über die Anwesenheit der Ammoniumkationen und/oder über die Anwesenheit der Carbonat-, beziehungsweise Bicarbonatanionen.

Wenn bei einer enzymatisch katalysierten Reaktion eine nicht ionogene Spezies in ionogene Bestandteile umgewandelt wird oder andererseits bei dieser enzymatisch katalysierten Reaktion ionogene Bestandteile in nicht-ionische Spezien umgewandelt werden, dann ist es auch möglich, die enzymatische Aktivität, beziehungsweise die Konzentration an Enzym in der wässrigen Probelösung zu bestimmen, indem man testet, mit welcher Geschwindigkeit die ionischen Spezien in der Probe gebildet werden oder aus der Probe entfernt werden.

Unter dem Ausdruck "Anion, das mit dem zu bestimmenden Kation formal ein Salz bildet", ist zu verstehen, dass dieses Anion nicht zwingendermassen das Hauptanion sein muss, welches in der im wesentlichen wässrigen Probelösung vorhanden ist. Verwendet man beispielsweise eine Testvorrichtung, die einen gegenüber Kaliumionen selektiven lipophilen Ionophor und ausserdem einen gegenüber Bicarbonationen selektiven Ionophor enthält, wobei mindestens einer dieser beiden Ionophore eine chromophore Gruppe aufweist, die in der Lage ist, ihre optischen Eigenschaften zu ändern, sobald dieser Ionophor mit dem entsprechenden Ion in Berührung kommt, dann kann diese Testvorrichtung zur Bestimmung der Kaliumionen und/oder der Bicarbonationen auch in einer solchen Probelösung herangezogen werden, welche wesentlich höhere molare Konzentrationen an Natriumchlorid enthält als an $K^+$ und $HCO_3^-$. Durch die erwähnte Testvorrichtung wird erreicht, dass selektiv eine simultane Coextraktion der Kaliumionen und der Bicarbonatanionen aus der wässrigen Probelösung in das Polymermaterial der Testvorrichtung erfolgt, während die Natriumionen und die Chloridanionen in der wässrigen Probelösung verbleiben.

Die Erfindung sei nun anhand von Beispielen näher erläutert.

Die nachfolgenden Beispiele dienen lediglich zur Erläuterung der Erfindung, sie sollen jedoch den Erfindungsgegenstand in keiner Weise einschränken.

Beispiel 1

Es wurde eine Testvorrichtung hergestellt, die als gegenüber Carbonationen selektivem Ionophor eine Verbindung der allgemeinen Formel Ia

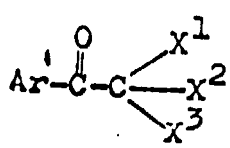

Ia

enthielt, in welcher

$X^1$, $X^2$ und $X^3$ Fluoratome sind und
Ar' ein äthersubstituierter Phenylrest der Formel

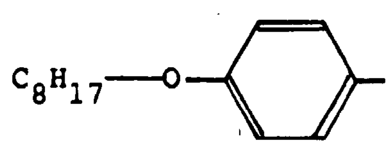

ist.

Des weiteren war in dieser Testvorrichtung als positiv geladener Ligand, dessen Ladungen mit Chloridionen elektrisch neutralisiert sind, das Methyltridodecylammoniumchlorid enthalten.

Zur Herstellung des gegenüber Carbonationen selektiven Teststreifens wurden die in der nachfolgenden Tabelle angegebenen Komponenten in den dort genannten Mengen verwendet:

Tabelle 1

| Bestandteil | Menge |
|---|---|
| Verbindung der Formel Ia | 3 mg |
| Methyltridodecylammoniumchlorid | 8 mg |
| Weichmacher | 74 mg |
| Polyvinylchlorid | 39,6 mg |
| Lösungsmittel | 23 ml |

Als Weichmacher wurde ein Dicarbonsäurediester, nämlich das Bis-2-äthylhexyl-sebacat verwendet und als Lösungsmittel Tetrahydrofuran eingesetzt.

Die Komponenten wurden in dem Lösungsmittel gelöst und anschliessend wurde die Mischung vergossen und das Lösungsmittel vollständig abdampfen gelassen. Man erhielt so einen nicht porösen Teststreifen, in dem die Komponenten in dem Kunststoffträgermaterial Polyvinylchlorid im wesentlichen gleichmässig verteilt waren.

Der nicht komplexierte carbonatselektive Ionophor der Formel Ia wies eine Absorption bei 298 nm auf. Sobald dieser Ionophor jedoch mit Carbonationen komplexiert war, verschwand diese Absorption bei 298 nm.

Bei der Bestimmung der Aktivität der Carbonationen in einer wässrigen Probelösung traten die Carbonatanionen der Probelösung aus der wässrigen Phase in die organische Phase des Teststreifens über und gleichzeitig wurde eine äquivalente Menge an Chloridanionen aus der organischen Phase des Teststreifens an die wässrige Probelösung nach dem folgenden Rekationsmechanismus freigesetzt.

$$CO_3^{2-}(aq) + 2\,Cl^-(org) \rightleftarrows CO_3^{2-}(compl.,org) + 2\,Cl^-(aq)$$

In Abhängigkeit von der Konzentration der Carbonationen in der wässrigen Probelösung erfolgte eine Gleichgewichtsverteilung der beiden ionischen Spezien Chloridanionen und Carbonatanionen zwischen der wässrigen Phase und der organischen Phase, wobei sich in der organischen Phase der Komplex aus dem Ionophor der Formel Ia und dem Carbonatanion bildete.

Während der Verwendung des Teststreifens blieb die Gesamtkonzentration aus freiem Ionophor der Formel I und mit Carbonationen komplexiertem Ionophor der Formel I in der organischen Phase konstant und diese Gesamtkonzentration aus komplexiertem und nicht komplexiertem Ionophor wird mit

$C_{L,tot}$ bezeichnet. Während des Tests trat also kein Ionophor aus der organischen Phase in die wässrige Phase über. Die Verteilung der Carbonatanionen zwischen der wässrigen Probelösung und der organischen Phase des Teststreifens entsprach also der folgenden Gleichung:

$$\frac{1-x}{2\,C_{L,tot}\,(C_{Q,tot}-(1-x)\,C_{L,tot})^2 \cdot x^2} = \frac{K\,a_{CO_3}}{(a_{Cl})^2} \qquad (1)$$

In dieser Gleichung haben die Symbole die folgende Bedeutung:

$C_{Q,tot}$ = Gesamtkonzentration an Methyltridodecylammoniumionen
X = relative Konzentration des unkomplexierten lipophilen Ionophors
$C_{L,tot}$ = Gesamtkonzentration an komplexiertem und nicht komplexiertem Ionophor
K = Gleichgewichtskonstante der dargestellten Ionenaustauschreaktion

Es gilt ferner:

$$x = \frac{C_{L'}}{C_{L,tot}}$$

$$K = \frac{\beta_2 \; k_{CO_3}}{(k_{Cl})^2}$$

wobei in diesen Formeln die Symbole die folgende Bedeutung besitzen:

$\beta_2$ = die Stabilitätskonstante des Komplexes zwischen Carbonationen und dem Ionophor L,
$K_{Cl}$ = Verteilungskoeffizient der Chloridionen zwischen der wässrigen Probelösung und der organischen Phase des Teststreifens

$$k_{CO_3} =$$

Verteilungskoeffizient der Carbonatanionen zwischen der wässrigen Phase der Probelösung und der organischen Phase des Teststreifens.

Die Absorption A bei der Wellenlänge von 298 nm ergibt sich dann aus der folgenden Gleichung:

$$A = A_1\, x + A_0\, (1 - x).$$

In dieser Gleichung bedeutet

$A_1$ = der Absorptionswert für x = 1 und
$A_0$ = der Absorptionswert für x = 0.

Die entsprechenden Messergebnisse werden durch Fig. 1 veranschaulicht.

In dieser Figur ist auf der Abszisse der Logarithmus des Aktivitätsverhältnisses von Carbonatanionen zu Chloridanionen in der wässrigen Probe aufgetragen, also der Wert

$$\log \frac{a_{CO_3^{2-}}}{(a_{Cl^-})^2} .$$

Auf der Ordinate ist die Absorption des Teststreifens bei 298 nm aufgetragen. Man sieht also, dass äquivalent dem Ansteigen des Konzentrationsverhältnisses von Carbonatanionen zu Chloridanionen in der wässrigen Phase die Absorption des Teststreifens bei der angegebenen Wellenlänge abnimmt.

<u>Beispiel 2</u>

Unter Verwendung des in Beispiel 1 beschriebenen für Carbonationen selektiven Ionophors und des dort beschriebenen Dicarbonsäurediesterweichmachers wurde eine ionenselektive Membran hergestellt, welche zusätzlich zu dem Tridodecyl-methyl-ammoniumchlorid auch noch die freie Base Tridodeylamin als für Wasserstoffionen selektiver Ionophor enthielt .

Der Teststreifen wurde unter Verwendung der in der nachfolgenden Tabelle angeführten Komponenten in den angegebenen Mengenverhältnissen hergestellt:

Tabelle II

| Bestandteil | Menge |
|---|---|
| Verbindung der Formel Ia | 3 mg |
| Methyltridodecylammoniumchlorid | 4 mg |
| Tridodecylamin | 3,8 mg |

Tabelle II (fortgesetzt)

| Bestandteil | Menge |
|---|---|
| Weichmacher | 74 mg |
| Polyvinylchlorid | 40,2 mg |
| Lösungsmittel | 25 ml |

Nach dem in Beispiel 1 beschriebenen Verfahren wurde unter Verwendung von Tetrahydrofuran als Lösungsmittel durch Vergiessen und Abdampfen des Lösungsmittels ein nicht poröser Teststreifen hergestellt.

Mit Hilfe dieses Teststreifens wurde in einer wässrigen Probelösung die Konzentration, beziehungsweise Aktivität an Bicarbonatanionen gemäss dem folgenden Reaktionsschema bestimmt:

$$HCO_3^-(aq) + Cl^-(org) \rightleftarrows H^+(compl.,org) + CO_3^{2-}(compl.,org) + Cl^-(aq)$$

Die Absorption dieses Teststreifens bei 298 nm ist dem Aktivitätsverhältnis von Bicarbonatanionen zu Chloridanionen in der wässrigen Probelösung äquivalent.

Die entsprechenden Messergebnisse sind in Fig. 2 dargestellt.

In der Fig. 2 ist auf der Abszisse der Logarithmus des Aktivitätsverhältnisses von Bicarbonatanionen zu Chloridanionen in der wässrigen Probelösung aufgetragen, also der Wert

$$\log \frac{a_{HCO_3^-}}{a_{Cl^-}}$$

Auf der Ordinate ist wieder die Absorption der Testvorrichtung bei 298 nm aufgetragen.

Man sieht aus der Fig. 2, dass äquivalent mit einem Anstieg des Konzentrationsverhältnisses von Bicarbonatanionen zu Chloridanionen in der wässrigen Testlösung die Absorption des Teststreifens bei der angegebenen Wellenlänge abnimmt.

Beispiel 3

Anhand dieses Beispiels wird die Herstellung eines Teststreifens zur Bestimmung der Aktivität, beziehungsweise Konzentration von Ammoniumionen und/oder Carbonationen in einer wässrigen Probelösung erläutert.

Als Ionophor, der gegenüber Carbonationen selektiv ist, verwendet man in der Testvorrichtung eine Verbindung der Formel Ib

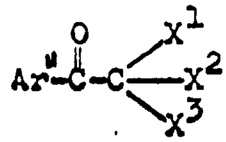

Ib

in welcher

$X^1$, $X^2$ und $X^3$ Fluoratome sind und

Ar" einen Rest der Formel

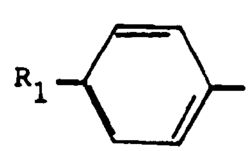

bedeutet, worin
$R_1$ die Gruppe $C_7H_{15}$-O-CO- ist.

Der Teststreifen enthielt ferner den aus dem Stande der Technik bekannten für Ammoniumionen selektiven lipophilen Ionophor auf Basis der Antibiotika Nonactin plus Monactin.

Des weiteren enthielt dieser Teststreifen den in Beispiel 1 angegebenen Weichmacher.

Der Teststreifen wurde unter Verwendung der in der nachfolgenden Tabelle angeführten Komponenten in den dort angegebenen Mengenverhältnissen hergestellt.

Tabelle III

| Bestandteil | Menge |
|---|---|
| Verbindung der Formel Ib | 5 mg |
| Nonactin/Monactin | 11,5 mg |
| Weichmacher | 70 mg |
| PVC | 37 mg |
| Lösungsmittel | 25 ml |

Es wurde ein weiterer Teststreifen hergestellt, der die gleiche Zusammensetzung aufwies, wie der oben genannte, jedoch zusätzlich noch 1,5 mg an Tetradodecylammonium-tetrakis(p-chlorphenyl)borat enthielt.

Die Herstellung der Teststreifen erfolgte nach dem in Beispiel 1 beschriebenen Verfahren durch Vergiessen und Abdampfen des verwendeten Lösungsmittels Tetrahydrofuran.

Man erhielt dabei nicht poröse Teststreifen. Bei der Testung der wässrigen Probelösung auf Carbonatanionen, beziehungsweise Ammoniumkationen trat eine Coextraktion der Ammoniumkationen mit den Carbonatanionen aus der wässrigen Phase in die organische Phase des Teststreifens nach dem folgenden Reaktionsmechanismus auf:

$$2\ NH_4^+(aq) + CO_3^{2-}\ (aq) \rightleftarrows 2\ NH_4^+(compl.,\ org) + CO_3^{2-}(compl.,org)$$

Die Absorption des für Carbonationen selektiven Esters der Formel Ib wurde bei 251 nm festgestellt und auch hier nahm die Absorption des Teststreifens bei steigender Komplexierung ab.

Mit diesem Teststreifen wird also das Produkt der Aktivität von Ammoniumionen und Carbonationen in der wässrigen Lösung, also der Wert

$$(a_{NH_4})^2 \cdot a_{CO_3}$$

bestimmt.

Die mit diesem Teststreifen erzielten Testergebnisse sind in Fig. 3 dargestellt.

In dieser Figur ist auf der Abszisse der Logarithmus des Produktes der Aktivität von Ammoniumionen und Carbonationen aufgetragen, also der Wert

$$\log\ [(a_{NH_4^+})^2 \cdot (a_{CO_3^{2-}})].$$

Auf der Ordinate ist wieder die Absorption des Teststreifens aufgetragen, diesmal aber gemessen bei 261 nm.

Aus der Fig. 3 ist die gute Aequivalenz der gemessenen Absorptionen mit den in der wässrigen Probelösung vorhandenen Konzentrationen ersichtlich.

## Beispiel 4

Anhand dieses Beispiels wird eine erfindungsgemässe Testvorrichtung gemäss der Variante B) erläutert, welche einen ersten kationselektiven lipophilen Ionophor enthält, der eine Selektivität für das zu bestimmende Kation aufweist und ferner einen zweiten kationselektiven lipophilen Ionophor enthält, der gegenüber Protonen selektiv ist, wobei die protonierte Form dieses zweiten kationselektiven Ionophors sich von der nicht protonierten Form bezüglich ihrer optischen Eigenschaften unterscheidet. Dieser zweite gegenüber Protonen selektive lipophile Ionophor ist also derjenige,

der eine chrompohore Gruppe trägt, während der gegenüber dem zu bestimmenden Kation selektive Ionophor frei von einer chromophoren Gruppe ist.

a) Herstellung des gegenüber Protonen selektiven lipophilen Ionophors

Der neue gegenüber Protonen selektive lipophile Ionophor weist die folgende Formel IIa

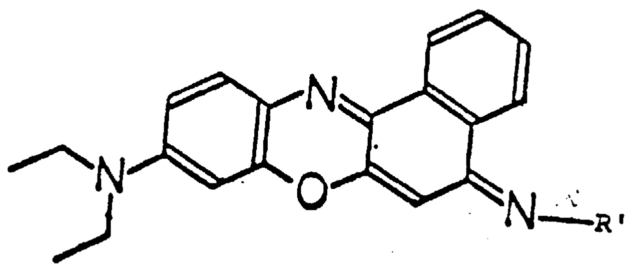

auf, wobei in dieser Formel

R' eine Gruppierung der Formel
-CO-R" ist, in welcher
R" ein geradkettiger Alyklrest der Formel
$-C_{17}H_{35}$ ist.

Die Herstellung dieses neuen gegenüber Protonen empfindlichen Chromoionophors der Formel IIa erfolgte, indem man Nilblau mit Stearinsäureanhydrid in Anwesenheit von Pyridin umsetzte.

Das Nilblau A ist in Form seines Chlorides von der Sigma Chemie GmbH in Deisenhofen, Bundesrepublik Deutschland käuflich erhältlich und dieses wurde durch Umsetzung mit Natronlauge in Wasser und Extraktion mit Methylenchlorid in die entsprechende freie Base der folgenden Formel

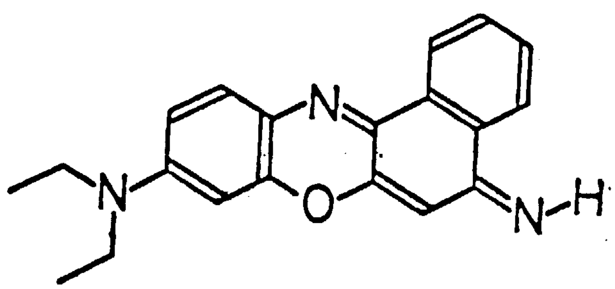

übergeführt.

Das Stearinsäureanhydrid wurde aus Stearinsäurechlorid (erhältlich von der Fluka AG, Buchs, Schweiz) und Calciumstearat (er hältlich von der Merck AG, in Tetrahydrofuran hergestellt.

Eine Mischung aus 300 mg (0,95 mmol) der basischen Form des Nilblaus der angegebenen Formel und 521 mg (0,95 mmol) an Stearinsäureanhydrid in 15 ml Pyridin wurde bei 80°C während 30 Minuten gerührt. Anschliessend wurde das Lösungsmittel abgedampft und der Rückstand in 50 ml Methylenchlorid gelöst. Diese organische Lösung wurde zweimal mit je 50 ml Wasser gewaschen. Anchliessend wurde die organische Phase filtriert, das Lösungsmittel abgedampft und der Rückstand durch eine Flash-Chromatographie auf Silicagel unter Verendung von Essigsäureäthylester als Elutionsmittel gereinigt. Man erhielt dabei 510 mg (0,87 mmol) des Endproduktes der Formel IIa. Die Ausbeute entsprach 91,5 % der theoretischen Ausbeute.

Die Struktur dieser neuen Verbindung der Formel IIa wurde durch das kernmagnetische Resonanzspektrum ($^1H$ NMR), aufgenommen in Deuterochlorophorm, das Infrarotspektrum, aufgenommen in Chlorophorm, sowie die Elementaranalyse bestätigt.

b) Herstellung einer Optodenmenbran zur optischen Bestimmung von Calciumionen

In dieser Membran wurde als gegenüber Calciumionen selektiver lipophiler neutraler Ionophor ein bekanntes Dicarbonsäurediamid eingesetzt, das seit einiger Zeit als calciumselektive Komponente in calciumselektiven Elektroden zur elektrometrischen Bestimmung von Calciumionen eingesetzt wurde. Dieser gegenüber Calciumionen selektive Ionophor war das (-)-(R,R,)-N,N'Di[(11-äthoxycarbonyl)undecyl]-N,N',4,5-tetramethyl-3,6-dioxaoctan-diamid.

Die Membran zur optischen Bestimmung der Konzentration, beziehungsweise Aktivität von Calciumionen in wässrigen Probelösungen wurde unter Verwendung der in der folgenden Tabelle IV angegebenen Komponenten in den dort angegebenen Mengen hergestellt:

Tabelle IV

| Bestandteil | Menge |
|---|---|
| Calciumselektives Dicarbonsäurediamid | 20 mg |
| Protonenselektive Komponente der Formel IIa | 5,5 mg |
| Negativ geladener mit Kaliumionen abgesättigter Ligand | 5,5 mg |
| Weichmacher | 146 mg |
| Polyvinylchlorid | 73 mg |

Als negativ geladener Ligand, dessen Ladungen durch Kaliumionen elektrisch neutralisiert sind, wurde das Kaliumtetrakis (p-chlorphenyl)borat verwendet.

Als Weichmacher diente ein Esterweichmacher, nämlich das Bis(2-äthylhexyl)sebacat.

Die in der Tabelle IV angegebenen Komponenten wurden in 1,5 ml frisch destilliertem Tetrahydrofuran gelöst. Diese Lösung wurde zu entsprechenden Membranen vergossen oder es wurden mit einer Spinnvorrichtung auf einer als Träger dienenden Glasplatte Membranen hergestellt, die eine genau einstellbare und gut reproduzierbare Dicke im Bereich von 0,5 Um bis 7 Um aufwiesen.

Vor der Durchführung der Messungen wurden die Membranen zehn Minuten in einer $10^{-3}$ molaren wässrigen Chlorwasserstoffsäure konditioniert und anschliessend nochmals zehn Minuten in einer 0,1 molaren Calciumchloridlösung.

Die Bestimmung der Calciumionen erfolgte in Testlösungen unterschiedlicher Gehalte an Calciumchlorid, bei denen der pH-Wert durch Verwendung von $10^{-3}$ molarer Zitronensäure und 0,1 molarer Natronlauge auf 6,2 eingestellt worden war.

Der neue gegenüber Protonen selektive Ionophor der Formel II weist in der mit Zitronensäure und Natronlauge auf 6,2 gepufferten, jedoch calciumfreien Testlösung ein Absorptionsmaximum bei 663 nm auf. Dieses Absorptionsmaximum der protonierten Form des neuen gegenüber Protonen empfindlichen lipophilen Chromoionophors verschwindet jedoch dann, wenn dieser Chromoionophor deprotoniert wird.

Wenn also die entsprechenden Membranen, die bei einer Wellenlänge von 663 nm eine Absorption zeigen, mit gepufferten Calciumchlorid enthaltenden Lösungen in Berührung gebracht werden, dann treten die Calciumionen aus der wässrigen Probelösung in die Polyvinylchloridmembran ein und sie werden von dem gegenüber Calciumionen selektiven Ionophor komplexiert. Aequivalente Mengen an Protonen treten von dem in der protonierten gefärbten Form vorliegenden Chromoionophor der Formel IIa aus der Polyvinylchloridmembran in die wässrige Probelösung aus. Dementsprechend nimmt mit steigenden Gehalten an Calciumionen in der Probelösung die Absorption der Membran bei der Wellenlänge von 663 nm ab.

Die entsprechenden Messungen wurden mit Calciumchloridlösungen einer Konzentration im Bereich von $10^{-6}$ molar bis $10^{-4}$ molar durchgeführt.

In Figur 4 sind die entsprechenden Messergebnisse veranschaulicht, wobei die Absorption in diesem Fall bei 660 nm gemessen wurde. Auf der Ordinate ist die Absorption aufgetragen und auf der Abszisse der Logarithmus der Calciumionenkonzentration der gepufferten Testlösungen.

Wie man sieht, nimmt die Absorption bei 660 nm äquivalent mit der ansteigenden Konzentration der Calciumionen in der Probelösung ab.

Beispiel 5

Nach dem gleichen Prinzip, wie in Beispiel 4 beschrieben und unter Verwendung des dort beschriebenen gegenüber Calciumionen selektiven lipophilen Ionophors wurde eine weitere Optodenmembran hergestellt.

In diesem Falle wurde jedoch als Chromionophor, der gegenüber Protonen empfindlich ist, eine ebenfalls neue Verbindung eingesetzt, die auch unter die allgemeine Formel II fällt. Diese neue Verbindung weist die folgende Formel

IIb

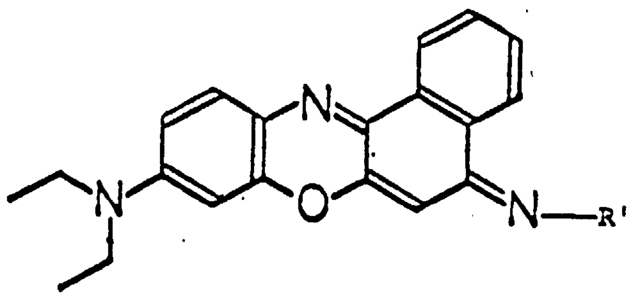

IIb

auf, wobei in dieser Formel der Rest R' eine Gruppierung der Formel

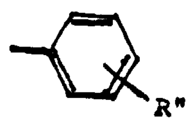

ist, in welcher der Rest
R" ein in der Parastellung gebundener geradkettiger Alkylrest der Formel
$-C_{16}H_{33}$
ist.

In der gleichen Weise, wie dies in Beispiel 4 beschrieben ist, jedoch unter Verwendung von o-Nitrophenyloctyläther als Weichmacher und unter Verwendung von Natrium-tetrakis[3,5-bis(trifluormethyl)phenyl]boratals mit Natriumionen elektrisch neutralisierter negativer Ligand wurde eine Polyvinylchloridmembran hergestellt. Auch in diesem Fall nahm die Absorption der entsprechenden Membranen bei einer Wellenlänge von etwa 660 nm proportional mit dem Gehalt der gepufferten Calciumchloridlösung ab.

Die entsprechenden Messungen wurden mit $10^{-5}$ molaren bis $10^{-1}$ molaren Calciumchloridlösungen durchgeführt.

Beispiel 6

Unter Verwendung des im Beispiel 4 beschriebenen neuen gegenüber Protonen selektiven Chromoionophors der Formel IIa wurden gegenüber Ammoniumionen empfindliche Membranen hergestellt.

Diese Membranen enthielten als gegenüber Ammoniumionen selektivem lipophilen Ionophor die im Beispiel 3 beschriebene Mischung aus Nonactin plus Monactin.

Die Membranen wurden unter Verwendung der in der folgenden Tabelle V angegebenen Bestandteile in den dort angegebenen Mengen hergestellt.

Tabelle V

| Bestandteil | Menge |
|---|---|
| Nonactin/Monactin | 6 mg |
| Verbindung der Formel IIa | 4 mg |
| Kalium-tetrakis(p-chlorphenyl)borat | 3,9 mg |
| Weichmacher | 160 mg |
| Polyvinylchlorid | 80 mg |

Als Weichmacher wurde der im Beispiel 4 verwemdete Esterweichmacher, nämlich das Bis(2-aethylhexyl)sebacat, eingesetzt. Die in der Tabelle V angegebenen Komponenten wurden in 1,5 ml frisch destilliertem Tetrahydrofuran gelöst und die Membranen wurden durch Vergiessen oder durch Verspinnen nach dem im Beispiel 4 beschriebenen Verfahren hergestellt.

Die Absorption dieser Membranen bei einer Wellenlänge von 660 nm nahm proportional mit dem Gehalt der wässrigen Probelösungen an Ammoniumchlorid ab.

Auch hier konnte eine sehr gute Aequivalenz des Absinkens der Absorption bei der angegebenen Wellenlänge mit dem Anstieg der Probelösungen an Ammoniumionen festgestellt werden. Mit Hilfe dieser Membranen war also das Verhältnis der Aktivität von Ammoniumionen zu Protonen in der Probelösung betimmbar, wobei die entsprechenden Messungen mit gepufferten $10^{-5}$ molaren bis $10^{-3}$ molaren wässrigen Lösungen an Ammoniumchlorid durchgeführt wurden.

Auch in diesem Falle zeigte es sich, dass äquivalent mit dem Eintreten von Ammoniumionen aus der wässrigen Probelösung in die Polyvinylchloridmembran der in der protonierten Form vorliegende neue gegenüber Protonen empfindliche Chromoionophor seine Protonen an die wässrige Probelösung abgab und dementsprechend in die nicht protonierte Form überging, die bei der Wellenlänge von 660 nm keine Absorption zeigte.

Beispiel 7

Unter Verwendung des im Beispiel 4 beschriebenen neuen gegenüber Protonen selektiven Chromoionophors der Formel IIa wurden weitere gegenüber Natriumionen, beziehungsweise gegenüber Kaliumionen selektive Membranen hergestellt. Diese Membranen enthielten ebenfalls als negativ geladenen Liganden, dessen negative Ladungen mit Kationen elektrisch neutralisiert sind, Alkalimetall-tetrakis(p-chlorphenyl)borate, und zwar die entsprechenden Natriumsalze, beziehungsweise Kaliumsalze.

Des weiteren enthielten diese Membranen als Weichmacher wieder einen Esterweichmacher, und zwar das Bis (2-äthylhexyl)sebacat.

Die entsprechenden gegenüber Kaliumionen selektiven Polyvinylchloridmembranen enthielten als kaliumselektiven lipophilen Ionophor Valinomycin.

Die gegenüber Natriumionen selektiven Polyvinylchloridmembranen enthielten als gegenüber Natriumionen selektivem lipophilem Ionophor entweder das N,N',N"-Triheptyl-N,N',N"-trimethyl-4,4',4"-propylidin-tris-(3-oxabutyramide) oder das N,N,N',N'-Tetracyclohexyl-1,2-phenylenedioxydiacetamide.

Auch in diesem Fall zeigten die entsprechenden Membranen bei der Testung von gepufferten Kaliumionen enthaltenden, beziehungsweise Natriumionen enthaltenden wässrigen Probelösungen ein Absinken der Absorption bei 660 nm mit zunehmenden Gehalt der entsprechenden Probelösungen an Natriumionen, beziehungsweise Kaliumionen.

**Patentansprüche**

1. Testvorrichtung zur optischen Bestimmung der Anwesenheit, beziehungsweise Konzentration, beziehungsweise Aktivität von Kationen, Anionen oder elektrisch neutralen ionogenen Spezien in einer im wesentlichen wässrigen Probelösung, wobei diese Testvorrichtung in einem im wesentlichen nicht porösen Trägermaterial, welches ein Polymermaterial ist, das nur geringe Anteile an hydrophilen Gruppen aufweist oder frei von hydrophilen Gruppen ist, mindestens einen kationselektiven lipophilen Ionophor und/oder mindestens einen anionselektiven lipophilen Ionophor enthält. dadurch gekennzeichnet, dass das Trägermaterial ausserdem eine weitere Komponente enthält. durch welche bei dem Kontakt der Testvorrichtung mit der wässrigen Probelösung ein simultaner Uebergang von Anionen und Kationen aus der wässrigen Probelösung in die Testvorrichtung und/oder ein Austausch von Kationen, beziehungsweise Anionen der Probelösung gegen in der Testvorrichtung enthaltene Kationen, beziehungsweise Anionen, beziehungsweise ein Austausch elektrisch neutraler ionogener Spezien der wässrigen Probelösung gegen elektrisch neutrale ionogene Spezien der Testvorrichtung gewährleistet ist, und wobei dieses Trägermaterial entweder

   A) mindestens einen kationselektiven lipophilen Ionophor und ausserdem mindestens einen anionselektiven lipophilen Ionophor enthält, wobei mindestens einer dieser Ionophore eine chromophore Gruppe aufweist, die in der Lage ist, ihre optischen Eigenschaften zu ändern, sobald dieser Ionophor mit Kationen, beziehungsweise Anionen, beziehungsweise elektrisch neutralen ionogenen Spezien der Probelösung in Berührung kommt oder
   B) zwei unterschiedliche kationselektive lipophile Ionophore in Kombination mit einem negativ geladenen Liganden und/oder negativ geladenen Stellen des Trägermaterials enthält, wobei diese negativen Ladungen durch Kationen elektrisch neutralisiert sind und/oder in Kombination mit mindestens einem Kationenaustauscher enthält, wobei mindestens einer der beiden kationselektiven lipophilen Ionophore eine chromophore Gruppe aufweist, die in der Lage ist, die optischen Eigenschaften des kationselektiven lipophilen Ionophoren zu ändern, sobald dieser mit Kationen oder elektrisch neutralen ionogenen Spezien der Probelösung in Berührung kommt, oder
   C) mindestens einen anionselektiven Ionophoren in Kombination mit mindestens einem positiv geladenen

Liganden und/oder positiv geladenen Stellen des Trägermaterials enthält, wobei diese positiven Ladungen mit Anionen elektrisch neutralisiert sind und/oder in Kombination mit mindestens einem Anionaustauscher enthält, wobei mindestens eine dieser Komponenten eine chromophore Gruppe aufweist, die in der Lage ist, die optischen Eigenschaften der Komponente zu ändern, sobald die Testvorrichtung mit Anionen, beziehungsweise elektrisch neutralen ionogenen Spezien der Probelösung in Berührung kommt.

**2.** Testvorrichtung gemäss Anspruch 1, dadurch gekennzeichnet, dass der eine chromophore Gruppe aufweisende lipophile Ionophor, beziehungsweise die eine chromophore Gruppe aufweisende Komponente eine solche ist, die in der Lage ist, eine Fluoreszenz auszubilden oder eine Fluoreszenzlöschung zu ergeben oder eine Veränderung der Lichtabsorption im ultravioletten Bereich, im sichtbaren Bereich oder im infraroten Bereich hervorzurufen, sobald der entsprechende Ionophor, beziehungsweise die entsprechende Komponente mit Kationen, Anionen oder elektrisch neutralen ionogenen Spezien der Probelösung in Berührung kommt.

**3.** Testvorrichtung gemäss Anspruch 1 oder 2, dadurch gekennzeichnet, dass sie eine Testvorrichtung

B) ist, wobei negativ geladene Liganden derselben und/oder negativ geladene Stellen des Trägermaterials durch andere Kationen elektrisch neutralisiert sind als durch Protonen.

**4.** Testvorrichtung gemäss einem der Ansprüche 1 bis 3, dadurch gekennzeichnet, dass sie

C) zwei unterschiedliche anionselektive lipophile Ionophore in Kombination mit mindestens einem positiv geladenen Liganden und/oder positiv geladenen Stellen des Trägermaterials enthält, wobei diese positiven Ladungen mit Anionen elektrisch neutralisiert sind und/oder in Kombination mit mindestens einem Anionenaustauscher enthält, wobei mindestens einer der beiden anionselektiven lipophilen Ionophore eine chromophore Gruppe aufweist, die in der Lage ist, die optischen Eigenschaften dieses Ionophoren zu ändern, sobald dieser mit Anionen oder elektrisch neutralen ionogenen Spezien der Probelösung in Berührung kommt.

**5.** Testvorrichtung gemäss Anspruch 1, dadurch gekennzeichnet, dass sie eine Testvorrichtung gemäss der Variante B) ist, die zwei unterschiedliche kationselektive lipophile Ionophore enthält, wobei der erste dieser unterschiedlichen kationselektiven lipophilen Ionophore keine chromophore Gruppe aufweist und der zweite unterschiedliche kationselektive lipophile Ionophor ein gegenüber Protonen selektiver lipophiler Chromoionophor ist, der seine optischen Eigenschaften ändert, sobald er von der protonierten Form in die nicht protonierte Form übergeht, und

dass die Testvorrichtung als negativ geladenen Liganden, dessen negative Ladungen durch Kationen elektrisch neutralisiert sind, vorzugsweise ein hydrophile Eigenschaften aufweisendes Borat enthält, insbesondere ein Alkalimetallsalz oder ein Ammoniumsalz eines Tetrakis(phenyl)borates mit gegebenenfalls 1, 2 oder 3 Substituenten in einem oder mehreren der Phenylkerne.

**6.** Testvorrichtung gemäss Anspruch 5, dadurch gekennzeichnet, dass sie als gegenüber Protonen selektivem lipophilem Ionophor eine neue Verbindung der folgenden Formel II

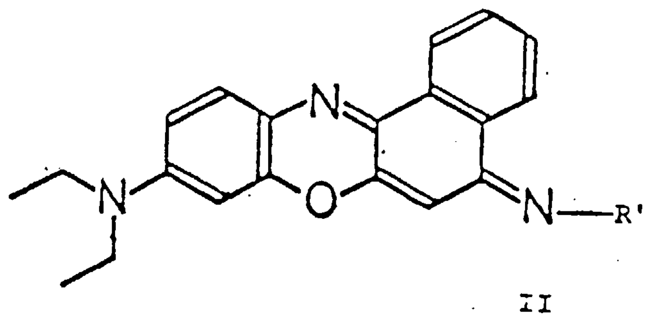

II

enthält, wobei in dieser Formel II R' ein mindestens 10 Kohlenstoffatome aufweisender aliphatischer Rest ist, der direkt an das Stickstoffatom oder über eine zweiwertige Gruppe, beispielsweise einen aromatischen Rest, einen cycloaliphatischen Rest, eine Gruppe der Formel -CO- oder -CS- gebunden ist und wobei dieser Rest R' vorzugsweise eine Gruppe der Formel
-CO-R"
oder

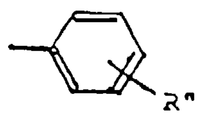

ist, in welcher
R" ein Alykylrest mit mindestens 10 Kohlenstoffatomen, vorzugsweise ein geradkettiger Alkylrest mit 14 - 26 Kohlenstoffatomen ist.

**7.** Testvorrichtung gemäss einem der Ansprüche 1 bis 4, dadurch gekennzeichnet, dass das nicht poröse Trägermaterial welches ein Polymermaterial, mit nur geringen Anteilen an hydrophilen Gruppen oder frei von hydrophilen Gruppen ist, aus der Klasse ausgewählt ist, die Polyvinylhalogenid- oder Polyvinylidenhalogenid- homopolymer oder -copolymer, umfasso und beispielsweise ein Polyvinylchloridhomopolymerisat oder ein Copolymerisat aus Polyvinylchlorid und geringeren Anteilen aus Polyvinylalkohol ist.

**8.** Testvorrichtung nach einem der Ansprüche 1 bis 7, dadurch gekennzeichnet, dass sie ein Teststreifen oder der selektive Bestandteil einer Optode zur Bestimmung von Anionen, Kationen, beziehungsweise elektrisch neutralen ionogenen Spezien in im wesentlichen wässrigen Probelösungen ist.

**9.** Testvorrichtung gemäss einem der Patentansprüche 1 bis 8, dadurch gekennzeichnet, dass sie mindestens einen anionselektiven lipophilen lonophor enthält, der eine chromophore Gruppe aufweist, wobei dieser anionselektive lonophor in der Lage ist, mit Anionen von Oxasäuren in Wechselwirkung zu treten und eine Carbonylverbindung ist, in welcher die Carbonylgruppe einen Teil eines Chromphoren darstellt, oder eine Carbonylverbindung ist. die eine fluoreszierende Gruppe aufweist, und wobei dann wenn diese Ketoverbindung mit einem Anion einer Oxasäure oder einer entsprechenden elektrisch neutralen ionogenen Spezies in Wechselwirkung tritt, eine Aenderung der Absorption im ultravioletten Bereich, im sichtbaren Bereich oder im infraroten Bereich hervorgerufen wird, oder wobei durch das Inwechselwirkungtreten der Ketoverbindung mit einem Anion einer Oxasäure, beziehungsweise einer entsprechenden elektrisch neutralen ionogenen Spezies eine Fluoreszenz hervorgerufen oder eine Fluoreszenz gelöscht wird.

**10.** Verfahren zur optischen Bestimmung der Anwesenheit, beziehungsweise Konzentration, beziehungsweise Aktivität von Kationen, Anionen oder elektrisch neutralen ionogenen Spezien in einer im wesentlichen wässrigen Probelösung, dadurch gekennzeichnet, dass man die wässrige Probelösung mit einer Testvorrichtung gemäss Anspruch 1 in Berührung bringt, wobei durch den Kontakt der Testvorrichtung mit der im wesentlichen wässrigen Probelösung ein simultaner Uebergang von Anionen und Kationen aus der wässrigen Probelösung in das Polymermaterial der Testvorrichtung und/oder ein Austausch von Kationen, beziehungsweise Anionen der Probelösung gegen in dem Polymermaterial der Testvorrichtung enthaltene Kationen, beziehungsweise Anionen, beziehungsweise ein Austausch elektrisch neutraler ionogener Spezien der wässrigen Probelösung gegen elektrisch neutrale ionogene Spezien des Polymermaterials der Testvorrichtung erfolgt, und wobei entweder

a) durch die Anwesenheit des zu bestimmenden Kations, eines mit diesem in Konkurrenz stehenden Kations oder eines Anions, das mit dem zu bestimmenden Kation formal ein Salz bildet, eine Fluoreszenz ausgebildet, eine Fluoreszenz gelöscht oder eine Veränderung der Lichtabsorption im ultravioletten Bereich, im sichtbaren Bereich oder im infraroten Bereich hervorgerufen wird oder
b) wobei durch die Anwesenheit des in der Probelösung zu bestimmenden Anions, eines mit diesem konkurrierenden Anions oder eines Kations, welches mit dem zu bestimmenden Anion formal ein Salz bildet, eine Fluoreszenz ausgebildet oder eine Fluoreszenz gelöscht oder eine Veränderung der Lichtabsorption im ultravioletten Bereich, im sichtbaren Bereich oder im infraroten Bereich hervorgerufen wird oder
c) dass durch die Berührung der Testvorrichtung mit einer in der Probelösung enthaltenen elektrisch neutralen ionogenen Spezies, beispielsweise einer Säure, einer Base, einem lonenassoziat oder einem Zwitterion, eine Fluoreszenz ausgebildet oder eine Fluoreszenz gelöscht oder eine Veränderung der Lichtabsorption im ultravioletten Bereich, im sichtbaren Bereich oder im infraroten Bereich hervorgerufen wird.

**11.** Verfahren nach Anspruch 10, dadurch gekennzeichnet, dass man das Verfahren mit einer Testvorrichtung A gemäss Patentanspruch 1 durchführt, welche in dem aus dem Polymer bestehenden Trägermaterial mindestens einen kationselektiven lipophilen lonophor und ausserdem mindestens einen anionselektiven lipophilen lonophor enthält, wobei mindestens einer dieser lonophoren eine chromophore Gruppe aufweist, welche die optischen Ei-

genschaften des Ionophor ändert, sobald dieser mit dem zu bestimmenden Kation, einem Anion, das mit diesem formal ein Salz bildet, dem zu bestimmenden Anion oder einem Kation, das mit diesem formal ein Salz bildet, beziehungsweise einer zu bestimmenden elektrisch neutralen ionogenen Spezies in Berührung kommt und

wobei durch die Anwesenheit des mindestens einen kationselektiven lipophilen Ionophors und des mindestens einen anionselektiven lipophilen Ionophors eine simultane Coextraktion von Kationen und Anionen oder eine Extraktion einer elektrisch neutralen ionogenen Spezies aus der wässrigen Probelösung in das Polymermaterial der Testvorrichtung erfolgt.

**12.** Verfahren nach einem der Ansprüche 10 bis 11, dadurch gekennzeichnet, dass man die Probelösung mit einer Testvorrichtung gemäss Anspruch 1 in Berührung bringt, die entweder

B) zwei unterschiedliche kationselektive lipophile Ionophore in Kombination mit einem negativ geladenen Liganden und/oder negativ geladenen Stellen des aus Polymermaterial bestehenden Trägermaterials enthält, wobei diese negativen Ladungen durch Kationen elektrisch neutralisiert sind und/oder in Kombination mit mindestens einem Kationenaustauscher enthält, wobei mindestens einer der beiden kationselektiven lipophilen Ionophore eine chromophore Gruppe aufweist, die in der Lage ist, die optischen Eigenschaften des kationselektiven lipophilen Ionophor zu ändern, sobald dieser mit Kationen oder einer elektrisch neutralen ionogenen Spezies der Probelösung in Berührung kommt und

wobei dann, wenn die Testvorrichtung mit der Probelösung in Berührung gebracht wird, das zu bestimmende Kation, beziehungsweise die zu bestimmende elektrisch neutrale ionogene Spezies aus der im wesentlichen wässrigen Probelösung in das Polymermaterial übergeht und dort an den für dieses Kation, beziehungsweise elektrisch neutrale ionogene Spezies, selektiven lipophilen Ionophor gebunden wird und gleichzeitig eine äquivalente Menge eines unterschiedlichen Kations, das in dem Polymermaterial der Testvorrichtung an den zweiten kationselektiven lipophilen Ionophor gebunden ist, aus diesem Polymermaterial in die wässrige Probelösung abgegebenen wird und wobei dann, wenn der kationselektive Ionophor, der für das zu bestimmende Kation, beziehungsweise die elektrisch neutrale ionogene Spezies selektiv ist. eine chromophore Gruppe aufweist, die Konzentration des zu bestimmenden Kations, beziehungsweise der elektrisch neutralen ionogenen Spezies, in der Probelösung durch eine Veränderung der optischen Eigenschaften dieses ionenselektiven lipophilen Ionophor feststellbar ist und dann, wenn der zweite kationselektive lipophile Ionophor derjenige ist, der eine chromophore Gruppe aufweist, die Konzentration des zu bestimmenden Kations, beziehungsweise der elektrisch neutralen ionogenen Spezies, in der Probelösung durch eine Aenderung der optischen Eigenschaften dieses zweiten kationselektiven lipophilen Ionophors durch die Freisetzung der anderen Kationenart an die Probelösung feststellbar ist oder

man die Probelösung mit einer Testvorrichtung gemäss Anspruch 4 C in Berührung bringt, die zwei unterschiedliche anionselektive lipophile Ionophore in Kombination mit mindestens einem positiv geladenen Liganden und/oder positiv geladenen Stellen des aus Polymermaterial aufgebauten Trägermaterials enthält, wobei diese positiven Ladungen mit Anionen elektrisch neutralisiert sind/oder in Kombination mit mindestens einem Anionenaustauscher enthält, wobei mindestens einer der beiden anionselektiven lipophilen Ionophore eine chromophore Gruppe aufweist, die in der Lage ist, die optischen Eigenschaften dieses Ionophor zu ändern, wenn dieser mit Anionen oder elektrisch neutralen ionogenen Spezien der Probelösung in Berührung kommt, und wobei das zu bestimmende Anion, beziehungsweise die elektrisch neutrale ionogene Spezies aus der Probelösung in die Testvorrichtung befördert und an den für diese Komponente selektiven lipophilen Ionophor gebunden wird und gleichzeitig eine äquivalente Menge eines Anions von dem zweiten lipophilen Ionophor an die wässrige Probelösung abgegeben wird und wobei dann, wenn der für das zu bestimmende Anion selektive lipophile Ionophor eine chromophore Gruppe aufweist, die Konzentration des zu bestimmenden Anions, beziehungsweise elektrisch neutralen ionogenen Spezies der Probelösung durch die Aenderung der optischen Eigenschaften dieses anionselektiven lipophilen Ionophor feststellbar ist und dann, wenn der für das zweite Anion selektive lipophile Ionophor eine chromophore Gruppe aufweist, die Konzentration des zu bestimmenden Anions, beziehungsweise der elektrisch neutralen ionogenen Spezies, durch die Aenderung der optischen Eigenschaften dieses Ionophor aufgrund der Freigabe des zweiten Anions an die Probelösung feststellbar ist.

**13.** Verfahren nach Anspruch 12, dadurch gekennzeichnet, dass man die Probelösung mit einer Testvorrichtung gemäss Anspruch 5 in Berührung bringt, die zwei unterschiedliche kationselektive lipophile Ionophore enthält, wobei der für das zu bestimmende Kation selektive Ionophor keine chromophore Gruppe aufweist und der die chromophore Gruppe tragende kationselektive lipophile Ionophor ein gegenüber Protonen selektiver Ionophor ist, der seine optischen Eigenschaften ändert, sobald er vom protonierten Zustand in den nicht protonierten Zustand übergeht, und wobei das Polymermaterial der Testvorrichtung zusätzlich zu den beiden unterschiedlichen kationselektiven lipophilen Ionophoren noch mindestens einen negativ geladenen Liganden und/oder negativ geladene Stellen des Polymermaterials enthält, wobei diese negativen Ladungen durch Kationen elektrisch neutralisiert sind und/oder das Polymermaterial der Testvorrichtung zusätzlich noch einen Kationenaustauscher enthält und wobei dann,

wenn diese Testvorrichtung mit einer Probelösung zusammengebracht wird, die das zu bestimmende Kation enthält, dieses Kation aus der wässrigen Probelösung in das Polymermaterial eintritt und dort von dem keine chromophore Gruppe aufweisenden für dieses Kation selektiven lipophilen Ionophor komplexiert wird und gleichzeitig eine äquivalente Menge von Protonen, die an den für Protonen selektiven eine chromophore Gruppe aufweisenden lipophilen Ionophor gebunden sind, von diesem an die wässrige Probelösung freigesetzt werden und wobei die Konzentration des zu bestimmenden Kations durch eine Aenderung der optischen Eigenschaften des gegenüber Protonen selektiven lipophilen Ionophors aufgrund seines Uebergangs von der protonierten Form in die nicht protonierte Form optisch feststellbar ist.

**14.** Verfahren nach einem der Ansprüche 10 bis 13, dadurch gekennzeichnet, dass in der Probelösung die Anwesenheit, beziehungsweise Konzentration, beziehungsweise Aktivität einer nicht-ionogenen Spezies bestimmt wird, indem man die nicht-ionogene Spezies durch eine Reaktion, beispielsweise eine enzymatisch katalysierte Reaktion, in mindestens ein Kation oder Anion oder eine elektrisch neutrale ionogene Spezies umwandelt und die Anwesenheit, beziehungsweise Konzentration, beziehungsweise Aktivität dieser Spezies mit der Testvorrichtung bestimmt.

**15.** Gegenüber Protonen selektiver lipophiler Chromoionophor, dadurch gekennzeichnet, dass er die folgende Formel II

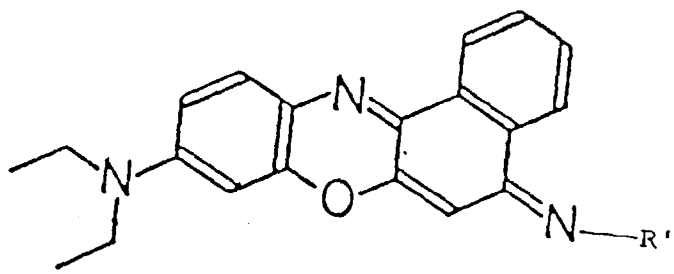

II

aufweist, wobei in dieser Formel

R' ein mindestens 10 Kohlenstoffatome aufweisender aliphatischer Rest ist, der direkt an das Stickstoffatom oder über eine zweiwertige Gruppe gebunden ist, vorzugsweise ein Rest der Formel
-CO-R"
oder

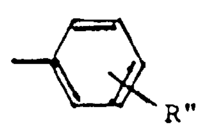

in welcher
R" ein vorzugsweise geradkettiger Alkylrest mit mindestens 10 Kohlenstoffatomen, insbesondere 14 - 26 Kohlenstoffatomen ist.

## Claims

**1.** Test apparatus for the optical determination of the presence or concentration or activity of cations, anions or electrically neutral ionogenic species in a substantially aqueous test solution, in which said test apparatus contains in a substantially non-porous carrier material, which is a polymer material that comprises only small proportions of hydrophilic groups or is free from hydrophilic groups, at least one cation-selective lipophilic ionophore and/or at

least one anion-selective lipophilic ionophore, **characterised in that** the carrier material contains in addition a further component by means of which, during the contact of the test apparatus with the aqueous test solution, a simultaneous migration of anions and cations out of the aqueous test solution into the test apparatus and/or an exchange of cations or anions of the test solution for cations or anions contained in the test apparatus, or an exchange of electrically neutral ionogenic species of the aqueous test solution for electrically neutral ionogenic species of the test apparatus is ensured, and in which said carrier material

A) contains at least one cation-selective lipophilic ionophore and in addition at least one anion-selective lipophilic ionophore, in which at least one of said ionophores comprises a chromophoric group which is capable of changing its optical properties as soon as said ionophore comes into contact with cations or anions or electrically neutral ionogenic species of the test solution or

B) contains two different cation-selective lipophilic ionophores in combination with a negatively charged ligand and/or negatively charged regions of the carrier material, in which said negative charges are electrically neutralized by cations, and/or contains them in combination with at leat one cation exchanger, in which at least one of the two cation-selective lipophilic ionophores comprises a chromophoric group which is capable of changing the optical properties of the cation-selective lipophilic ionophore as soon as the latter comes into contact with cations or electrically neutral ionogenic species of the test solution, or

C) contains at least one anion-selective ionophore in combination with at least one positively charged ligand and/or positively charged regions of the carrier material, in which said positive charges are electrically neutralized with anions, and/or contains them in combination with at least one anion exchanger, in which at least one of said components comprises a chromophoric group which is capable of changing the optical properties of the component as soon as the test apparatus comes into contact with anions or electrically neutral ionogenic species of the test solution.

**2.** Test apparatus according to claim 1, **characterised in that** the lipophilic ionophore comprising a chromophoric group or the component comprising a chromophoric group is one that is capable of forming a fluorescence or producing a fluorescence extinction or causing a change of the light absorption in the ultraviolet range, in the visible range or in the infrared range as soon as the corresponding ionophore or the corresponding component comes into contact with cations, anions or electrically neutral ionogenic species of the test solution.

**3.** Test apparatus according to claim 1 or claim 2, **characterised in that** it is a test apparatus

B), in which negatively charged ligands of the latter and/or negatively charged regions of the carrier material are electrically neutralized by cations other than protons.

**4.** Test apparatus according to any one of claims 1 to 3, **characterised in that** it

C) contains two different anion-selective lipophilic ionophores in combination with at least one positively charged ligand and/or positively charged regions of the carrier material, in which said positive charges are neutralized electrically with anions, and/or contains them in combination with at least one anion exchanger, in which at least one of the two anion-selective lipophilic ionophores comprises a chromophoric group which is capable of changing the optical properties of said ionophore as soon as the latter comes into contact with anions or electrically neutral ionogenic species of the test solution.

**5.** Test apparatus according to claim 1, **characterised in that** it is a test apparatus according to variant B) which contains two different cation-selective lipophilic ionophores, in which the first of said different cation-selective lipophilic ionophores does not comprise a chromophoric group and the second different cation-selective lipophilic ionophore is a lipophilic chromoionophore selective in respect of protons, which changes its optical properties as soon as it passes from the protonated form into the non-protonated form, and that the test apparatus contains, as a negatively charged ligand whose negative charges are neutralized electrically by cations, preferably a borate possessing hydrophilic properties, in particular an alkali metal salt or an ammonium salt of a tetrakis(phenyl)borate with, as necessary, 1, 2 or 3 substituents in one or more of the phenyl nuclei.

**6.** Test apparatus according to claim 5, **characterised in that** it contains as a lipophilic ionophore selective in respect of protons a new compound of the following formula II

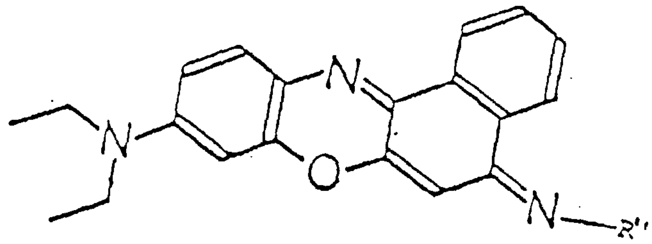

where in this formula II R' is an aliphatic residue comprising at least 10 carbon atoms, which is bonded directly to the nitrogen atom or via a divalent group, for example an aromatic residue, a cycloaliphatic residue or a group with the formula -CO- or -CS-, and where said residue R' is preferably a group with the formula

-CO-R"

or

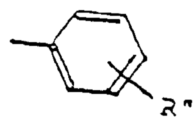

in which

R" is an alkyl residue with at least 10 carbon atoms, preferably a straight-chain alkyl residue with 14 - 26 carbon atoms.

**7.** Test apparatus according to any one of claims 1 to 4, **characterised in that** the non-porous carrier material, which is a polymer material with only small proportions of hydrophilic groups or is free from hydrophilic groups, is selected from the class which embraces polyvinyl halide or polyvinylidene halide homopolymers or copolymers and is for example a polyvinyl chloride homopolymer or a copolymer consisting of polyvinyl chloride and smaller proportions of polyvinyl alcohol.

**8.** Test apparatus according to any one of claims 1 to 7, **characterised in that** it is a test strip or the selective component of an optode for the determination of anions, cations or electrically neutral ionogenic species in substantially aqueous test solutions.

**9.** Test apparatus according to any one of claims 1 to 8, **characterised in that** it contains at least one anion-selective lipophilic ionophore which comprises a chromophoric group, in which said anion-selective ionophore is capable of interacting with anions of oxa-acids and is a carbonyl compound in which the carbonyl group represents a part of a chromophore, or is a carbonyl compound which comprises a fluorescent group, and in which, if said keto compound interacts with an anion of an oxa-acid or a corresponding electrically neutral ionogenic species, a change of the absorption in the ultraviolet spectrum, in the visible spectrum or in the infrared spectrum is caused, or in which a fluorescence is caused or a fluorescence is extinguished due to the interactions of the keto compound with an anion of an oxa-acid or a corresponding electrically neutral ionogenic species.

**10.** Method for the optical determination of the presence or concentration or activity of cations, anions or electrically neutral ionogenic species in a substantially aqueous test solution, **characterised in that** the aqueous test solution is brought into contact with a test apparatus according to claim 1, in which by the contact of the test apparatus with the substantially aqueous test solution a simultaneous migration of anions and cations out of the aqueous test solution into the polymer material of the test apparatus and/or an exchange of cations or anions of the test solution for cations or anions contained in the polymer material of the test apparatus, or an exchange of electrically neutral ionogenic species of the aqueous test solution for electrically neutral ionogenic species of the polymer material of the test apparatus takes place, and in which either

a) due to the presence of the cation to be determined, of a cation competing with the latter or of an anion which theoretically forms a salt with the cation to be determined a fluorescence is formed, a fluorescence is extinguished or a change of the light absorption in the ultraviolet spectrum, in the visible spectrum or in the infrared spectrum is caused or

b) due to the presence of the anion to be determined in the test solution, of an anion competing with the latter or of a cation which theoretically forms a salt with the anion to be determined, a fluorescence is formed, a fluorescence is extinguished or a change of the light absorption in the ultraviolet spectrum, in the visible spectrum or in the infra-red spectrum is caused or

c) that due to the contact of the test apparatus with an electrically neutral ionogenic species contained in the test solution, for example an acid, a base, an ion association complex or a zwitterion, a fluorescence is formed or a fluorescence is extinguished or a change of the light absorption in the ultraviolet spectrum, in the visible spectrum or in the infra-red spectrum is caused.

**11.** Method according to claim 10, **characterised in that** the method is carried out with a test apparatus A according to claim 1 which contains in the carrier material consisting of polymer material at least one cation-selective lipophilic ionophore and in addition at least one anion-selective lipophilic ionophore, in which at least one of said ionophores comprises a chromophoric group which changes the optical properties of the ionophore as soon as the latter comes into contact with the cation to be determined, an anion which theoretically forms a salt with the latter, the anion to be determined or a cation which theoretically forms a salt with the latter, or with an electrically neutral ionogenic species to be determined, and in which a simultaneous co-extraction of cations and anions or an extraction of an electrically neutral ionogenic species out of the aqueous test solution into the polymer material of the test apparatus takes place due to the presence of the at least one cation-selective lipophilic ionophore and the at least one anion-selective lipophilic ionophore.

**12.** Method according to any one of claims 10 to 11, **characterised in that** the test solution is brought into contact with a test apparatus according to claim 1 which either

B) contains two different cation-selective lipophilic ionophores in combination with a negatively charged ligand and/or negatively charged regions of the carrier material consisting of polymer material, in which said negative charges are neutralized electrically by cations, and/or contains them in combination with at least one cation exchanger, in which at least one of the two cation-selective lipophilic ionophores comprises a chromophoric group which is capable of changing the optical properties of the cation-selective lipophilic ionophore as soon as the latter comes into contact with cations or an electrically neutral ionogenic species of the test solution and in which, if the test apparatus is brought into contact with the test solution, the cation to be determined, or the electrically neutral ionogenic species to be determined, migrates out of the substantially aqueous test solution into the polymer material and is there bonded to the lipophilic ionophore selective for said cation or electrically neutral ionogenic species, and simultaneously an equivalent amount of a different cation, which is bonded in the polymer material of the test apparatus to the second cation-selective lipophilic ionophore, is transferred out of said polymer material into the aqueous test solution, and in which, if the cation-selective ionophore which is selective for the cation to be determined, or the electrically neutral ionogenic species, comprises a chromophoric group, the concentration of the cation to be determined, or of the electrically neutral ionogenic species, is ascertainable in the test solution through a change in the optical properties of said ion- selective lipophilic ionophore and if the second cation-selective lipophilic ionophore is the one which comprises a chromophoric group, the concentration of the cation to be determined, or of the electrically neutral ionogenic species, is ascertainable in the test solution through a change in the optical properties of said second cation-selective lipophilic ionophore due to the release of the other kind of cation to the test solution or

the test solution is brought into contact with a test apparatus according to claim 4 C which contains two different anion-selective lipophilic ionophores in combination with at least one positively charged ligand and/or positively charged regions of the carrier material consisting of polymer material, in which said positive charges are neutralized electrically with anions, or contains them in combination with at least one anion exchanger, in which at least one of the two anion-selective lipophilic ionophores comprises a chromophoric group which is capable of changing the optical properties of said ionophore if the latter comes into contact with anions or electrically neutral ionogenic species of the test solution, and in which the anion to be determined, or the electrically neutral ionogenic species, is conveyed out of the test solution into the test apparatus and is bonded to the lipophilic ionophore selective for said component and simultaneously an equivalent amount of an anion is transferred from the second lipophilic ionophore to the aqueous test solution, and in which, if the lipophilic ionophore selective for the anion to be determined comprises a chromophoric group, the concentration of the anion to be determined, or electrically neutral ionogenic species of the test solution, is ascertainable through the change in the optical properties of said anion-selective lipophilic ionophore, and if the lipophilic ionophore selective for the second anion comprises a chromophoric group, the concentration of the anion to be determined, or of the electrically neutral ionogenic species, is ascertainable through the change in the optical properties of said ionophore by virtue of the release of the second anion to the test solution.

**13.** Method according to claim 12, **characterised in that** the test solution is brought into contact with a test apparatus according to claim 5 which contains two different cation-selective lipophilic ionophores, in which the ionophore selective for the cation to be determined does not comprise a chromophoric group and the cation-selective lipophilic ionophore bearing the chromophoric group is an ionophore selective in respect of protons which changes its optical properties as soon as it passes from the protonated state into the non-protonated state, and in which the polymer material of the test apparatus contains in addition to the two different cation-selective lipophilic ionophores also at least one negatively charged ligand and/or negatively charged regions of the polymer material, in which said negative charges are neutralized electrically by cations and/or the polymer material of the test apparatus contains in addition also a cation exchanger and in which, if said test apparatus is brought into contact with a test solution which contains the cation to be determined, said cation migrates out of the aqueous test solution into the polymer material and is there complexed by the lipophilic ionophore selective for said cation and not comprising a chromophoric group and at the same time an equivalent amount of protons which are bonded to the lipophilic ionophore selective in respect of protons and comprising a chromophoric group are liberated by said lipophilic ionophore to the aqueous test solution, and in which the concentration of the cation to be determined is ascertainable optically through a change in the optical properties of the lipophilic ionophore selective in respect of protons by virtue of its passage from the protonated form into the non-protonated form.

**14.** Method according to any one of claims 10 to 13, **characterised in that** in the test solution the presence or concentration or activity of a non-ionogenic species is determined by the non-ionogenic species being converted by a reaction, for example an enzymatically catalysed reaction, into at least one cation or anion or an electrically neutral ionogenic species and the presence or concentration or activity of said species is determined with the test apparatus.

**15.** Lipophilic chromoionophore selective in respect of protons, **characterised in that** it exhibits the following formula II

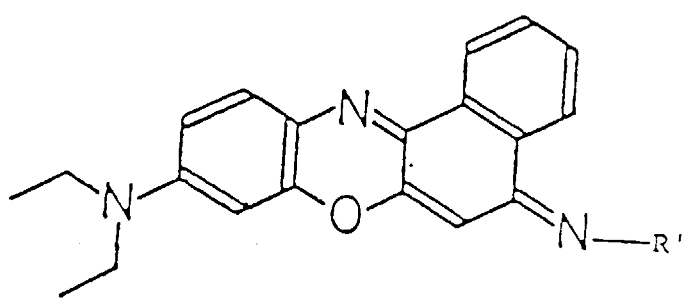

II

where in this formula,

R' is an aliphatic residue comprising at least 10 carbon atoms which is bonded to the nitrogen atom directly or via a divalent group, preferably a residue with the formula
-CO-R"
or

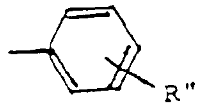

in which
R" is a preferably straight-chain alkyl residue with at least 10 carbon atoms, in particular 14 - 26 carbon atoms.

## Revendications

1. Dispositif de test pour la détermination optique de la présence, de la concentration ou encore de l'activité de cations, d'anions, d'espèces ionogènes électriquement neutres dans une solution d'échantillon essentiellement aqueuse, ce dispositif de test contenant au moins un ionophore lipophile sélecteur de cations et/ou au moins un ionophore lipophile sélecteur d'anions dans un support essentiellement non poreux qui est un matériau polymère présentant seulement une faible proportion en radicaux hydrophiles ou tout à fait dépourvu de radicaux hydrophiles, caractérisé en ce que le matériau de support contient par ailleurs un autre composant qui garantit, lors du contact du dispositif de test avec la solution d'échantillon aqueuse, un transfert simultané d'anions et cations de la solution d'échantillon aqueuse dans le dispositif de test et/ou un échange de cations ou d'anions dans la solution d'échantillon contre les cations ou anions contenus dans le dispositif de test ou encore un échange d'espèces ionogènes électriquement neutres de la solution d'échantillon aqueuse contre des espèces ionogènes électriquement neutres du dispositif de test, ce support contenant soit

   A) au moins un ionophore lipophile sélecteur de cations et en outre au moins un ionophore lipophile sélecteur d'anions, au moins un de ces ionophores présentant un radical chromophore qui est en mesure de modifier ses propriétés optiques dès que cet ionophore entre en contact avec des cations, des anions ou des espèces ionogènes électriquement neutres de la solution d'échantillon ou

   B) deux ionophores lipophiles sélecteurs de cations différents en combinaison avec un ligand chargé négativement et/ou des zones chargées négativement du matériau de support, ces charges négatives étant neutralisées électriquement par des cations et/ou en combinaison avec au moins un échangeur de cations, au moins un des deux ionophores lipophiles sélecteurs de cations présentant un radical chromophore qui est en mesure de modifier les propriétés optiques de l'ionophore lipophile sélecteur de cations dès que celui-ci entre en contact avec des cations ou des espèces ionogènes électriquement neutres de la solution d'échantillon, ou

   C) au moins un ionophore sélecteur d'anions en combinaison avec au moins un ligand chargé positivement et/ou des zones chargées positivement du matériau de support, ces charges positives étant neutralisées électriquement avec des anions et/ou en combinaison avec au moins un échangeur d'anions, au moins un de ces composants présentant un radical chromophore qui est en mesure de modifier les propriétés optiques des composants dès que le dispositif de test entre en contact avec des anions ou des espèces ionogènes électriquement neutres de la solution d'échantillon.

2. Dispositif de test selon la revendication 1 caractérisé en ce que l'ionophore lipophile présentant un radical chromophore ou le composant présentant un radical chromophore est en mesure de former une fluorescence, de faire disparaître une fluorescence ou d'entraîner une modification de l'absorption de la lumière dans la gamme ultraviolette, dans la gamme visible ou dans la gamme infrarouge dès que l'ionophore correspondant ou le composant correspondant entre en contact avec des cations, des anions ou des espèces ionogènes électriquement neutres de la solution d'échantillon.

3. Dispositif de test selon la revendication 1 ou 2 caractérisé en ce qu'il est un dispositif de test
   B) dont les ligands chargés négativement et/ou les zones chargées négativement du matériau de support sont neutralisés électriquement par d'autres cations que des protons.

4. Dispositif de test selon une des revendications 1 à 3 caractérisé en ce qu'il contient soit
   C) deux ionophores lipophiles sélecteurs d'anions différents en combinaison avec au moins un ligand chargé positivement et/ou des zones chargées positivement du matériau de support, ces charges positives étant neutralisées électriquement par des anions et/ou en combinaison avec au moins un échangeur d'anions, au moins un des deux ionophores lipophiles sélecteurs d'anions présentant un radical chromophore qui est en mesure de modifier les propriétés optiques de cet ionophore dès que celui-ci entre en contact avec les anions ou les espèces ionogènes électriquement neutres de la solution d'échantillon.

5. Dispositif de test selon la revendication 1, caractérisé en ce qu'il est un dispositif de test selon la variante B) qui contient deux ionophores lipophiles sélecteurs de cations différents, le premier de ces ionophores lipophiles sélecteurs de cations différents ne présentant pas de radical chromophore et le deuxième étant un chromo-ionophore lipophile sélecteur de protons qui modifie ses propriétés optiques dès qu'il passe de la forme protonisée en une forme non protonisée et
   en ce que le dispositif de test contient de préférence un borate présentant des propriétés hydrophiles comme

ligand chargé négativement dont les charges négatives sont neutralisées électriquement par des cations, en particulier un sel de métal alcalin ou un sel d'ammonium d'un tétrakis(phényl)borate avec éventuellement 1, 2 ou 3 substituants dans un ou plusieurs des noyaux phényle.

**6.** Dispositif de test selon la revendication 5 caractérisé en ce qu'il contient comme ionophore lipophile sélecteur de protons un nouveau composé de formule II suivante

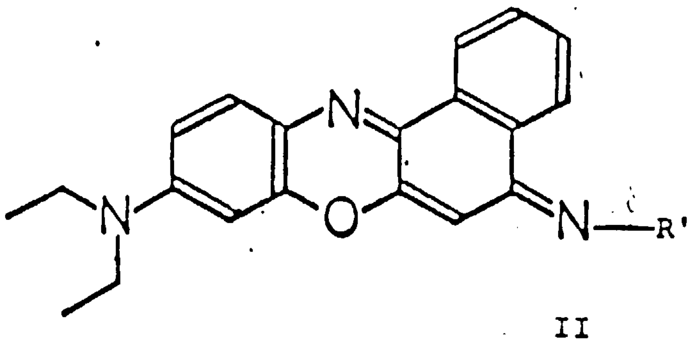

II

dans laquelle R' désigne un radical aliphatique contenant au moins 10 atomes de carbone qui est lié directement à l'atome d'azote ou par l'intermédiaire d'un radical divalent, par exemple un radical aromatique, un radical cycloaliphatique, un radical de formule -C0- ou -CS-, ce radical R' étant de préférence un groupe de formule

-CO-R"

ou

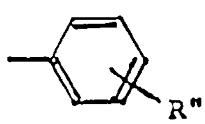

dans laquelle R" est un radical alkyle avec au moins 10 atomes de carbone, de préférence un radical alkyle à chaîne droite avec 14 à 26 atomes de carbone.

**7.** Dispositif de test selon une des revendications 1 à 4 caractérisé en ce que le matériau de support non poreux est un matériau polymère avec seulement une faible proportion en radicaux hydrophiles ou tout à fait dépourvu de radicaux hydrophiles qui est choisi parmi les homopolymères ou copolymères d'halogénure de polyvinyle ou d'halogénure de polyvinylidène et est par exemple un homopolymère de chlorure de polyvinyle ou un copolymère de chlorure de polyvinyle et une faible proportion d'alcool polyvinylique.

**8.** Dispositif de test selon une des revendications 1 à 7 caractérisé en ce qu'il est une bande de test ou le constituant sélecteur d'une optode pour la détermination d'anions, de cations ou d'espèces ionogènes électriquement neutres dans des solutions d'échantillon essentiellement aqueuses.

**9.** Dispositif de test selon une des revendications 1 à 8 caractérisé en ce qu'il contient au moins un ionophore lipophile sélecteur d'anions qui présente un radical chromophore, cet ionophore sélecteur d'anions étant en mesure d'interagir avec les anions d'oxyacide et est un composé carbonyle dans lequel le radical carbonyle constitue une partie d'un chromophore ou un composé carbonyle qui présente un radical fluorescent et où lorsque ce composé cétonique interagit avec un anion d'un oxyacide ou d'une espèce ionogène électriquement neutre correspondante, une modification de l'absorption dans la gamme ultraviolette, dans la gamme visible ou dans la gamme infrarouge est provoquée ou une interaction du composé cétonique avec un anion d'un oxyacide ou d'une espèce ionogène électriquement neutre correspondante provoque une fluorescence ou la disparition de cette fluorescence.

**10.** Procédé de détermination optique de la présence, de la concentration ou encore de l'activité de cations, d'anions ou d'espèces ionogènes électriquement neutres dans une solution d'échantillon essentiellement aqueuse, caractérisé en ce que la solution d'échantillon aqueuse est amenée à entrer en contact avec un dispositif de test selon la revendication 1, le contact du dispositif de test avec la solution d'échantillon essentiellement aqueuse entraînant un transfert simultané d'anions et de cations de la solution d'échantillon aqueuse dans le matériau polymère du dispositif de test et/ou un échange entre les cations ou anions de la solution d'échantillon et les cations ou anions contenus dans le matériau polymère du dispositif de test ou un échange d'espèces ionogènes électriquement neutres de la solution d'échantillon aqueuse contre les espèces ionogènes électriquement neutres du matériau polymère du dispositif de test et où

a) la présence du cation à déterminer, d'un cation en concurrence avec lui ou d'un anion qui forme formellement un sel avec le cation à déterminer provoque une fluorescence, fait disparaître une fluorescence ou entraîne une modification de l'absorption de la lumière dans la gamme ultraviolette, dans la gamme visible ou dans la gamme infrarouge ou

b) la présence dans la solution d'échantillon de l'anion à déterminer, d'un anion en concurrence avec lui ou d'un cation qui forme formellement un sel avec l'anion à déterminer, provoque une fluorescence, la disparition d'une fluorescence ou la modification de l'absorption de la lumière dans la gamme ultraviolette, dans la gamme visible ou dans la gamme infrarouge ou

c) le contact du dispositif de test avec une espèce ionogène électriquement neutre contenue dans la solution d'échantillon, par exemple un acide, une base, une association d'ions ou un zwitterion provoque la formation d'une fluorescence, la disparition d'une fluorescence ou une modification de l'absorption de la lumière dans la gamme ultraviolette, dans la gamme visible ou dans la gamme infrarouge.

**11.** Procédé selon la revendication 10 caractérisé en ce que le procédé est exécuté avec un dispositif de test a selon la revendication 1 qui contient dans le support composé d'un matériau polymère, au moins un ionophore lipophile sélecteur de cations et, en outre, au moins un ionophore lipophile sélecteur d'anions, au moins un de ces ionophores présentant un radical chromophore qui modifie les propriétés optiques de l'ionophore dès que celui-ci entre en contact avec le cation à déterminer, avec un anion qui forme formellement un sel avec celui-ci, avec l'anion à déterminer ou avec une espèce ionogène électriquement neutre à déterminer et,

la présence d'au moins un ionophore lipophile sélecteur de cations et d'au moins un ionophore lipophile sélecteur d'anions entraînant une coextraction simultanée de cations et d'anions ou une extraction d'une espèce ionogène électriquement neutre de la solution d'échantillon aqueuse dans le matériau polymère du dispositif de test.

**12.** Procédé selon une des revendications 10 à 11 caractérisé en ce que la solution d'échantillon est mise en contact avec un dispositif de test selon la revendication 1 qui contient soit

b) deux ionophores lipophiles sélecteurs de cations différents en combinaison avec un ligand chargé négativement et/ou des zones chargées négativement du matériau de support composé d'un polymère, ces charges négatives étant neutralisées électriquement par des cations et/ou en combinaison avec au moins un échangeur de cations, au moins un des deux ionophores lipophiles sélecteurs de cations présentant un radical chromophore qui est en mesure de modifier les propriétés optiques de l'ionophore lipophile sélecteur de cations dès que celui-ci entre en contact avec des cations ou une espèce ionogène électriquement neutre de la solution d'échantillon et où

lorsque le dispositif de test est mis en contact avec la solution d'échantillon, le cation à déterminer ou l'espèce ionogène électriquement neutre à déterminer passe de la solution d'échantillon essentiellement aqueuse dans le matériau polymère et est lié à cet endroit par l'ionophore liphophile sélecteur de ce cation ou de cette espèce ionogène électriquement neutre et une quantité équivalente d'un cation différent qui est lié dans le matériau polymère du dispositif de test au deuxième ionophore lipophile sélecteur de cations est libérée du matériau polymère dans la solution d'échantillon aqueuse et où, lorsque l'ionophore sélecteur de cations qui est sélecteur pour le cation à déterminer ou l'espèce ionogène électriquement neutre à déterminer présente un radical chromophore qui permet de déterminer la concentration du cation à déterminer ou de l'espèce ionogène électriquement neutre dans la solution d'échantillon par une modification des propriétés optiques de cet ionophore lipophile sélecteur d'ions, lorsque le deuxième ionophore lipophile sélecteur de cations présente un radical chromophore qui permet de déterminer la concentration des cations à déterminer ou de l'espèce ionogène électrique neutre dans la solution d'échantillon par une modification des propriétés optiques de ce deuxième ionophore lipophile sélecteur de cations par la libération de l'autre type de cation dans la solution d'échantillon ou la solution d'échantillon est mise en

contact avec un dispositif de test selon la revendication 4 C), les deux ionophores lipophiles sélecteurs d'anions différents en combinaison avec au moins un ligand chargé positivement et/ou des zones chargées positivement du support constitué du matériau polymère, ces charges positives étant neutralisées électriquement par des anions et/ou en combinaison avec au moins un échangeur d'anions, au moins un des deux ionophores lipophiles sélecteurs d'anions présentant un radical chromophore qui est en mesure de modifier les propriétés optiques de cet ionophore lorsque celui-ci entre en contact avec des anions, des espèces ionogènes électriquement neutres dans la solution d'échantillon et où l'anion à déterminer ou l'espèce ionogène électriquement neutre passe de la solution d'échantillon dans le dispositif de test et est lié à l'ionophore lipophile sélecteur de ce composant et une quantité équivalente d'un anion est libéré du deuxième ionophore lipophile dans la solution d'échantillon aqueuse simultanément et où, lorsque l'ionophore lipophile sélecteur de l'anion à déterminer présente un radical chromophore, la concentration de l'anion à déterminer ou de l'espèce ionogène électriquement neutre de la solution d'échantillon peut être déterminée par la modification des propriétés optiques de cet ionophore lipophile sélecteur d'anions et où, lorsque l'ionophore lipophile sélecteur du deuxième anion présente un radical chromophore, la concentration de l'anion à déterminer ou de l'espèce ionogène électriquement neutre peut être déterminée par la modification des propriétés optique de cet ionophore en raison de la libération du deuxième anion dans la solution d'échantillon.

**13.** Procédé selon la revendication 12 caractérisé en ce que la solution d'échantillon est mise en contact avec le dispositif de test selon la revendication 5 qui contient deux ionophores lipophiles sélecteurs de cations différents, l'ionophore sélecteur du cation à déterminer ne présentant pas de radical chromophore et l'ionophore lipophile sélecteur de cations portant le radical chromophore étant un ionophore sélecteur de protons qui modifie ses propriétés optiques dès qu'il passe de la forme protonisée à une forme non protonisée et où le matériau polymère du dispositif de test contient en plus des deux ionophores lipophiles sélecteurs de cations différents, au moins un ligand chargé négativement et/ou des zones chargées négativement du matériau polymère, ces charges négatives étant neutralisées électriquement par des cations et/ou le matériau polymère du dispositif de test contient encore un échangeur de cations et où, lorsque ce dispositif de test est amené à une solution d'échantillon, qui contient le cation à déterminer, ce cation passe de la solution d'échantillon aqueuse dans le matériau polymère et y est complexé par l'ionophore lipophile sélecteur de ce cation ne présentant pas de radical chromophore et une quantité équivalente de protons qui sont liés à l'ionophore lipophile présentant un radical chromophore et sélecteur de protons est simultanément libérée de celui-ci dans la solution d'échantillon aqueuse, la concentration du cation à déterminer pouvant être déterminée par la modification des propriétés optiques de l'ionophore lipophile sélecteur de protons en raison de son passage d'une forme protonisée à une forme non protonisée.

**14.** Procédé selon une des revendications 10 à 13 caractérisé en ce que la présence, la concentration ou encore l'activité d'une espèce non ionogène dans la solution d'échantillon est déterminée en transformant l'espèce non ionogène par une réaction, par exemple par une réaction catalysée enzymatiquement, en au moins un cation ou un anion ou une espèce ionogène électriquement neutre, et en ce que la présence, la concentration ou encore l'activité de cette espèce peut être déterminée par le dispositif de test.

**15.** Chromo-ionophore liphophile sélecteur de protons caractérisé en ce qu'il présente la formule suivante II

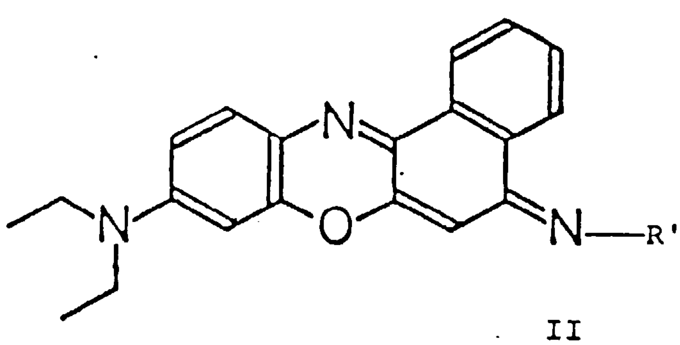

II

dans laquelle R' désigne un radical aliphatique présentant au moins 10 atomes de carbone qui est lié à l'atome d'azote directement ou par l'intermédiaire d'un radical divalent, de préférence un radical de formule

-CO-R"

ou

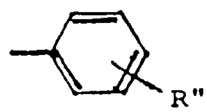

dans laquelle

R" désigne de préférence un radical alkyle à chaîne droite avec au moins 10 atomes de carbone, en particulier 14 à 16 atomes de carbone.

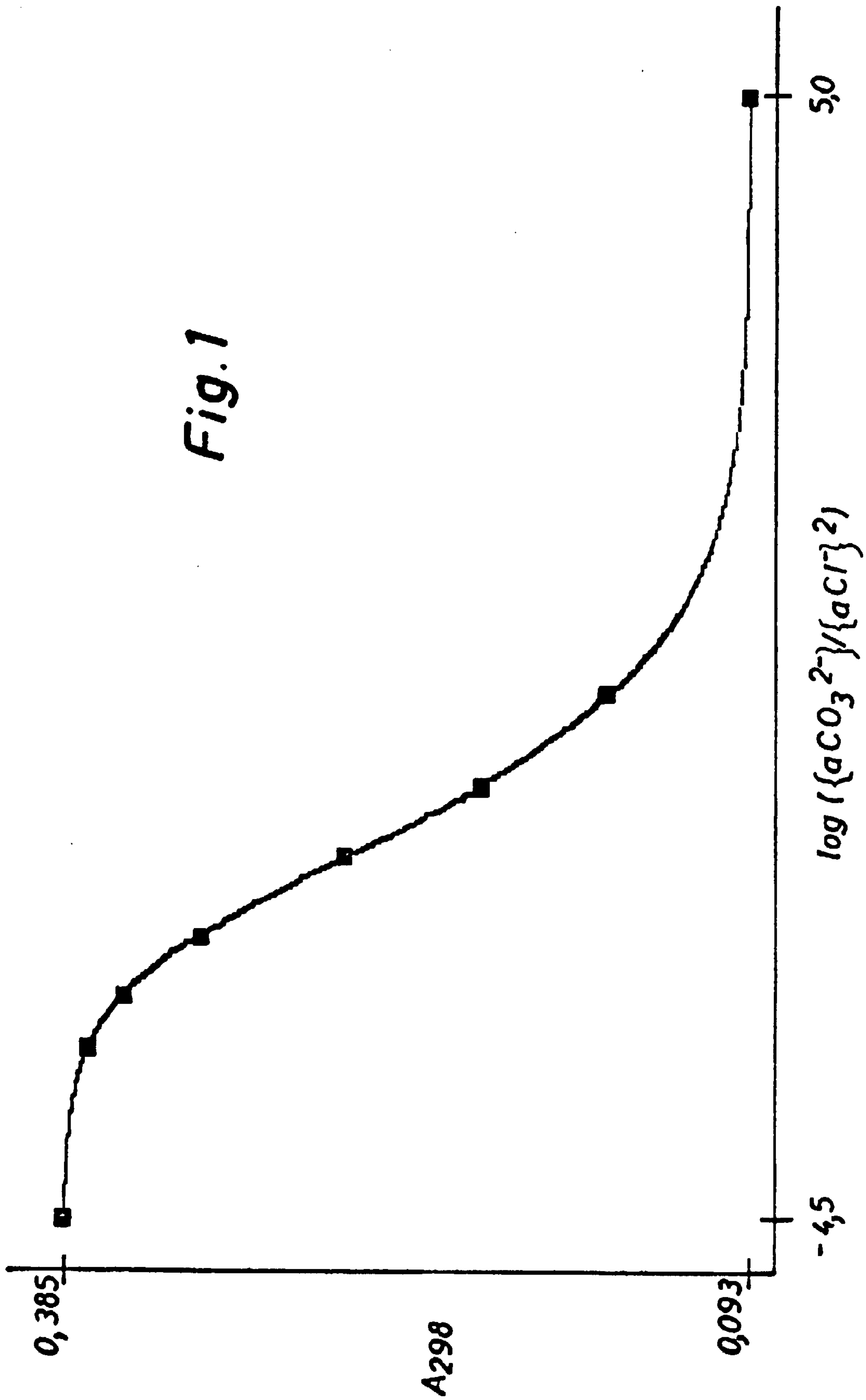


Fig.1

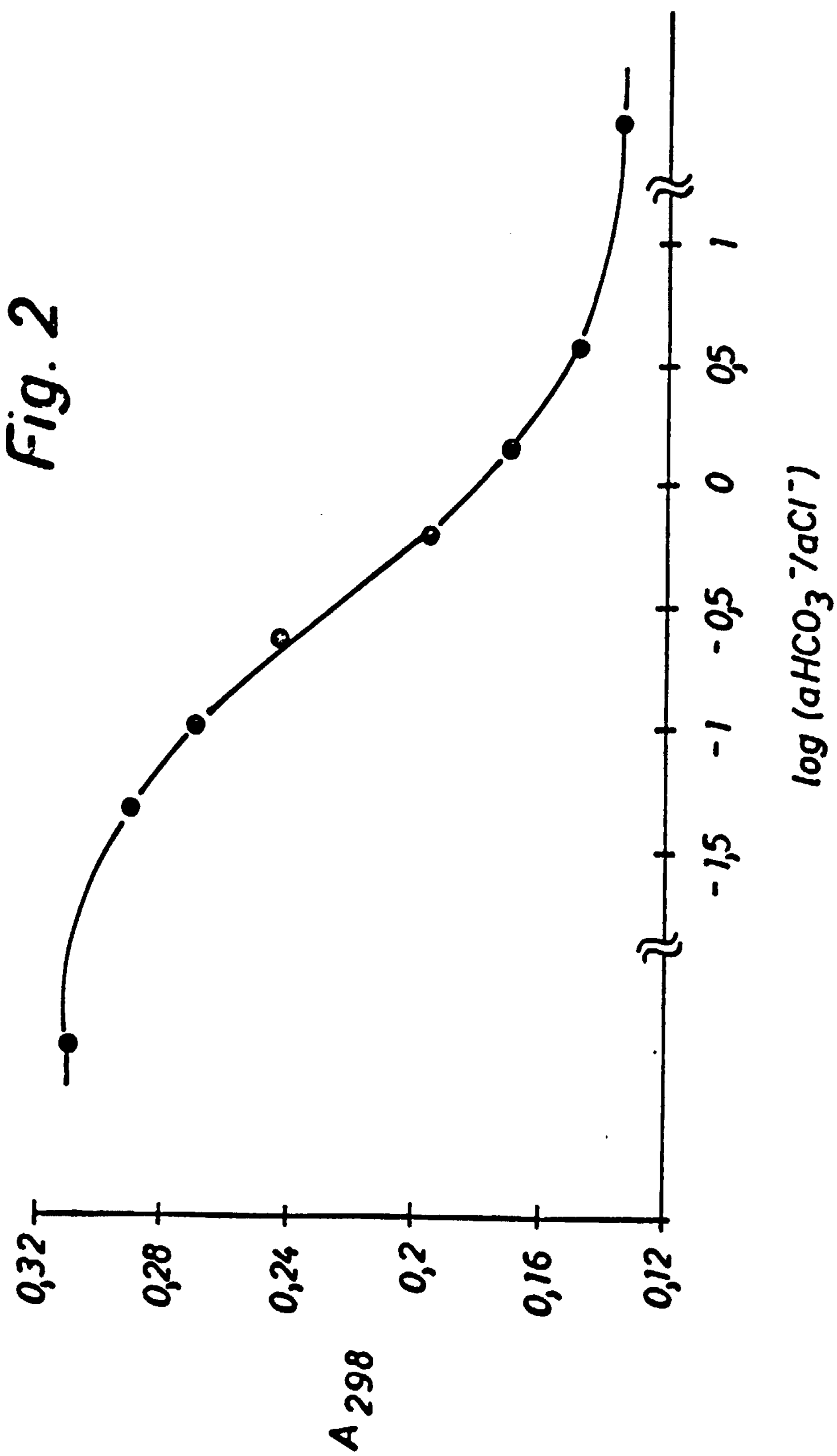


Fig. 2

Fig. 3

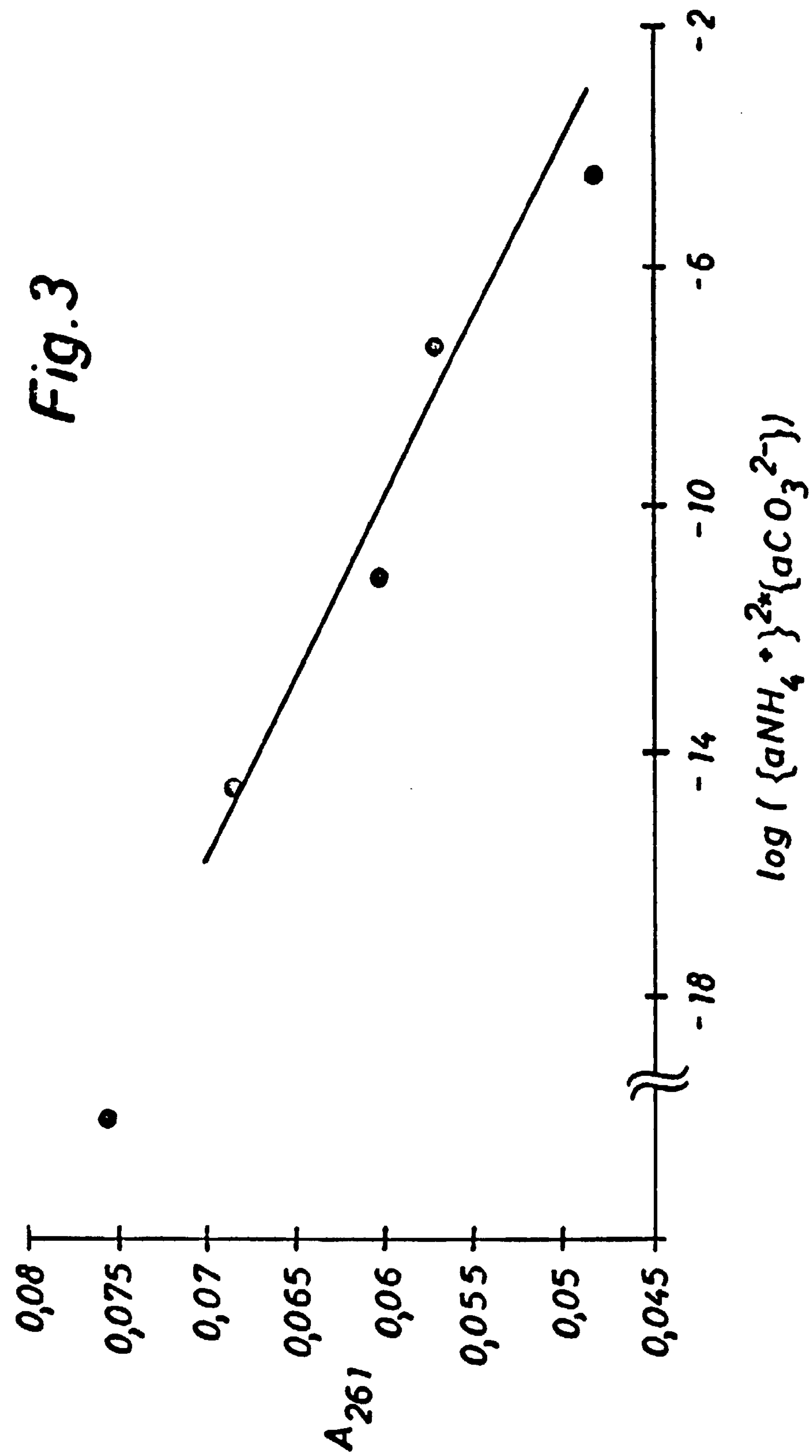

0,08
0,075
0,07
0,065
0,06
0,055
0,05
0,045
A261
-18
-14
-10
-6
-2
log ( {aNH4+}2*{aCO32-})

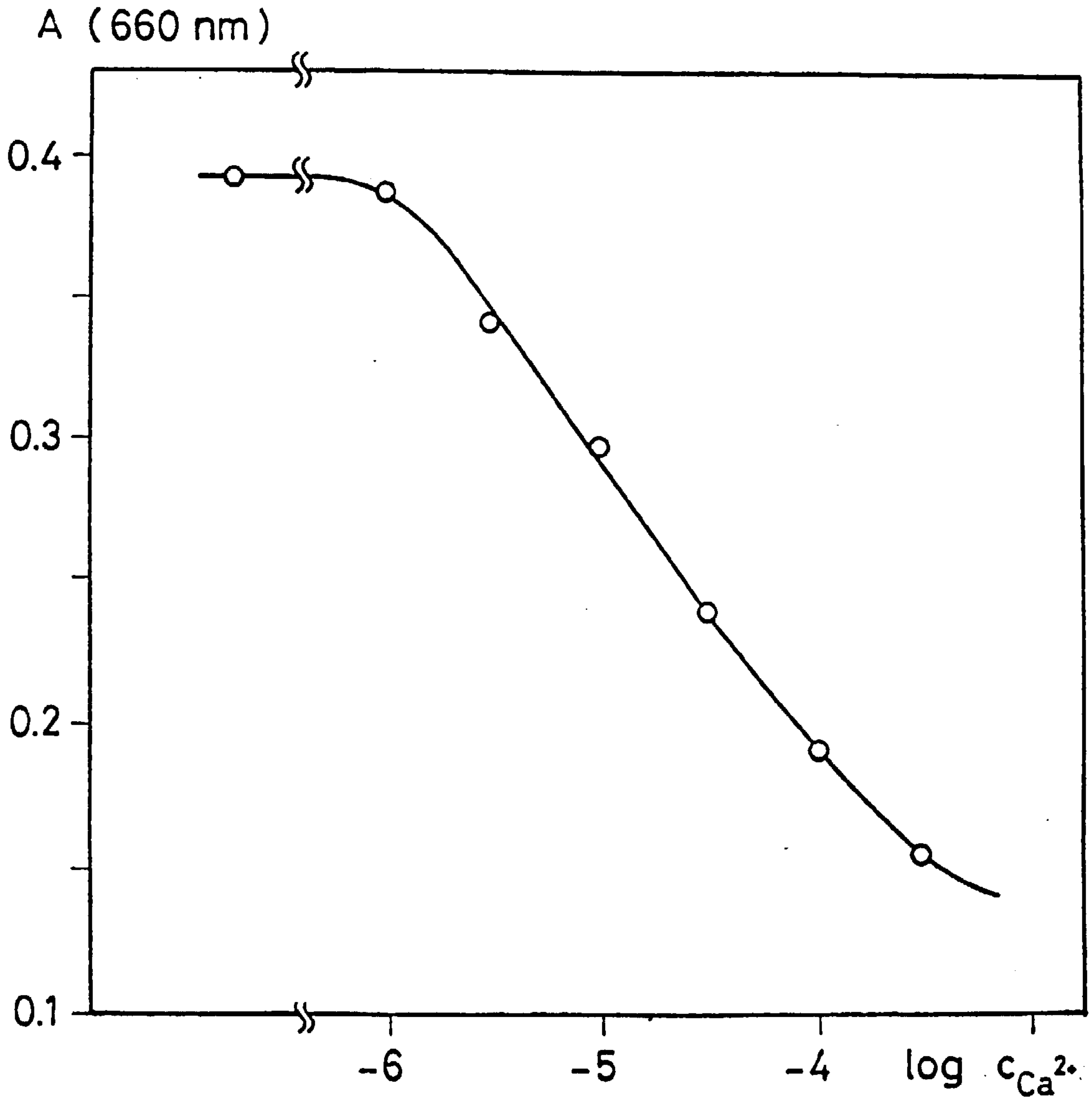


Fig. 4